(19) Europäisches Patentamt
European Patent Office
Office européen des brevets

(11) Veröffentlichungsnummer: **0 028 011 B1**

(12) **EUROPÄISCHE PATENTSCHRIFT**

(45) Veröffentlichungstag der Patentschrift: **20.11.86**

(21) Anmeldenummer: **80106483.3**

(22) Anmeldetag: **23.10.80**

(51) Int. Cl.[4]: **C 07 C 103/46,** C 07 C 103/737, C 07 D 307/24, C 07 D 249/08, C 07 D 233/60, C 07 D 307/64, C 07 C 125/065, C 07 C 155/02, C 07 C 155/08, A 01 N 47/12, A 01 N 47/14

(54) **Homoserin-Derivate, Verfahren zu ihrer Herstellung und ihre Verwendung als Mikrobizide.**

(43) Veröffentlichungstag der Anmeldung:
**06.05.81 Patentblatt 81/18**

(45) Bekanntmachung des Hinweises auf die Patenterteilung:
**20.11.86 Patentblatt 86/47**

(84) Benannte Vertragsstaaten:
**AT BE CH DE FR GB IT LI NL SE**

(56) Entgegenhaltungen:
**DE - A - 2 513 732**
**DE - A - 2 515 091**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Kunz, Walter, Dr., Im Goldbrunnen 55, CH-4104 Oberwil (CH)**
Erfinder: **Eckhardt, Wolfgang, Dr., Breslauerstrasse 16, D-7850 Lörrach (DE)**
Erfinder: **Hubele, Adolf, Dr., Obere Egg 9, CH-4465 Magden (CH)**
Erfinder: **Riebli, Peter, Dittingerstrasse 12, CH-4053 Basel (CH)**

(74) Vertreter: **Zumstein, Fritz, Dr. et al, Bräuhausstrasse 4, D-8000 München 2 (DE)**

**EP 0 028 011 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Homoserin-Derivate der Formel I, ihre Herstellung und ihre Verwendung als Mikrobizide

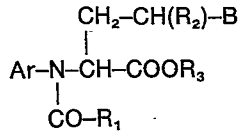 (I),

worin $R_1$ eine gegebenenfalls durch ein Sauerstoffatom unterbrochene aliphatische Kette aus 2 bis 6 Kohlenstoffatomen bedeutet, oder für eine gegebenenfalls durch Halogen substituierte 2-Furyl-, 2-Tetrahydrofuryl-, 1H-1,2,4-Triazolylmethyl-, 1-Imidazolylmethyl-, 1-Pyrazolylmethyl-, $C_2$–$C_4$-Alkenyl- oder Cyclopropylgruppe steht oder worin, wenn B für Halogen steht, $R_1$ eine Halogenmethylgruppe bedeutet; $R_2$ für Wasserstoff oder Methyl steht, $R_3$ für Wasserstoff oder $C_1$–$C_4$-Alkyl steht und Ar

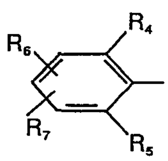 oder 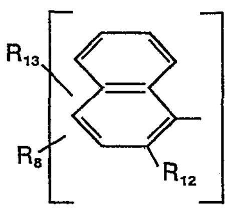 bedeutet,

wobei $R_4$ für $C_1$–$C_3$-Alkoxkyl, $C_1$–$C_3$-Alkoxy oder Halogen, $R_5$ für $C_1$–$C_3$-Alkyl, $C_1$–$C_3$-Alkoxy, $NH_2$, Halogen oder $NO_2$, $R_6$ für Wasserstoff, $NO_2$, $NH_2$, $C_1$–$C_3$-Alkyl, $C_1$–$C_3$-Alkoxy oder Halogen, $R_7$ und $R_8$ für Wasserstoff oder Methyl stehen, $R_{12}$ Methyl, $NO_2$ oder $NH_2$ darstellt, $R_{13}$ für Wasserstoff, Methyl, $NO_2$ oder $NH_2$ steht und B eine der Gruppen–XH,–$XR_9$,

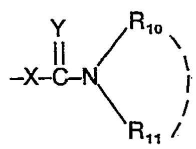 oder

Halogen bedeutet, wobei X und Y unabhängig voneinander für Sauerstoff oder Schwefel stehen; $R_9$ für eine gegebenenfalls durch Halogen, $C_1$–$C_3$-Alkoxy oder $C_1$–$C_3$-Alkylthio substituierte $C_1$–$C_5$-Alkylgruppe steht, $R_{10}$ Wasserstoff, Methyl oder Ethyl bedeutet, $R_{11}$ für eine gegebenenfalls durch Halogen substituierte $C_1$–$C_5$-Alkylgruppe oder für einen gegebenenfalls durch Halogen, Methyl, Trifluormethyl oder Nitro substituierten Phenylrest steht oder $R_{10}$ und $R_{11}$ zusammen mit dem Stickstoffatom, an das sie gebunden sind, einen Imidazol- oder 1,2,4-Triazolring bilden.

Die vorliegende Erfindung betrifft auch Verbindungen der Formel I, ihre Herstellung und ihre Verwendung als Mikrobizide, worin Ar

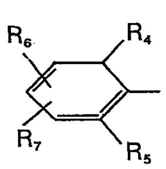 oder 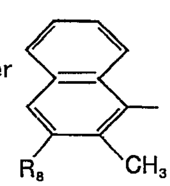 bedeutet,

$R_1$, $R_2$, $R_3$, $R_4$ und B die vorstehend genannte Bedeutung haben und $R_5$ für $C_1$–$C_3$-Alkyl, $C_1$–$C_3$-Alkoxy oder Halogen, $R_6$ für Wasserstoff, $C_1$–$C_3$-Alkyl, $C_1$–$C_3$-Alkoxy oder Halogen und $R_7$ und $R_8$ für Wasserstoff oder Methyl stehen.

Unter Alkyl oder Alkylanteil eines anderen Substituenten sind je nach Zahl der angegebenen C-Atome beispielsweise folgende Gruppen zu verstehen: Methyl, Ethyl, Propyl, Butyl, Pentyl oder Hexyl sowie deren Isomere, wie z.B. Isopropyl, Isobutyl, tert.-Butyl, Isopentyl usw.

Alkenyl steht beispielsweise für Vinyl, 1-Propenyl, Allyl, 1-Butenyl, 2-Butenyl usw.

Mit diesen beispielhaften Aufzählungen ist keine Limitierung verbunden.

Halogen steht für Fluor, Chlor, Brom oder Jod, vorzugsweise für Chlor, Brom oder Jod.

Verbindungen der Formel I sind sehr wertvolle Wirkstoffe gegen schädliche Mikroorganismen.

Es werden solche mikrobiziden Wirkstoffe der Formel I bevorzugt, deren Substituenten folgende Gruppen repräsentieren:

Bei $R_1$:

a) Halogenmethyl, $C_1$–$C_3$-Alkoxymethyl, $C_2$–$C_3$-Alkenyl, Cyclopropyl, 2-Furyl-, 2-Tetrahydrofuryl, 1-H-1,2,4-Triazolylmethyl, 1-Imidazolylmethyl

b) Halogenmethyl, $C_1$–$C_2$-Alkoxymethyl, $C_2$–$C_3$-Alkenyl, 2-Furyl, 2-Tetrahydrofuryl

Bei $R_2$: Wasserstoff, Methyl,

Bei $R_3$: Wasserstoff, $C_1$–$C_3$-Alkyl

Bei $R_4$: Methyl, Ethyl, Cl

Bei $R_5$: Methyl, Ethyl, Methoxy, Cl

Bei $R_6$:

a) Wasserstoff, Methyl, Ethyl, Methoxy, Cl, Br, $NH_2$, $NO_2$,

b) Wasserstoff, 3-methyl, 3-Ethyl, 3-Cl, 4-Cl, 3-Methoxy, 3-$NO_2$, 3-$NH_2$

Bei $R_7$: Wasserstoff, Methyl

Bei $R_8$:

a) Wasserstoff, Methyl,

b) Wasserstoff, 3-Methyl,

Bei $R_{12}$: $CH_3$, $NO_2$

Bei $R_{13}$: Wasserstoff, $NO_2$

Bei B: Hydroxy, Chlor, Brom, Jod
oder wenn B: –XH, –$XR_9$,

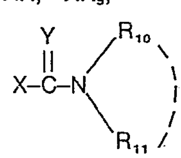

Bei X und Y: Sauerstoff

Bei $R_9$: $C_1$–$C_3$-Alkyl

Bei $R_{10}$: Wasserstoff, Methyl, Ethyl

Bei $R_{11}$:

a) gegebenenfalls durch Halogen substituiertes $C_1$–$C_3$-Alkyl oder Phenyl
b) $C_1$–$C_3$-Alkyl, Phenyl

Bei 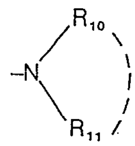 :

1,2,4-Triazolyl, Imidazolyl

Nachfolgende Gruppen von Verbindungen der Formel I werden bevorzugt:

Eine bevorzugte Gruppe bilden substituierte Phenyl-Verbindungen der Formel I (Ar = substituiertes Phenyl), worin $R_1$ für $C_1$–$C_3$-Alkoxymethyl, 2-Furyl oder 2-Tetrahydrofuryl steht, $R_2$ und $R_3$ Wasserstoff bedeuten, B für OH oder SH, $R_4$ für $C_1$–$C_3$-Alkyl, $C_1$–$C_3$-Alkoxy oder Halogen, $R_5$ für $C_1$–$C_3$-Alkyl, $C_1$–$C_3$-Alkoxy, Halogen oder Nitro, $R_6$ für Wasserstoff oder $C_1$–$C_3$-Alkyl stehen und $R_7$ Wasserstoff oder Methyl bedeutet; diese bevorzugte Gruppe soll als Untergruppe Ia bezeichnet werden.

Bevorzugt wird ferner die Gruppe von substituierten Phenyl-Verbindungen der Formel I, worin $R_1$ für $C_1$–$C_3$-Alkoxymethyl, 2-Furyl oder 2-Tetrahydrofuryl steht, $R_2$ Wasserstoff bedeutet, $R_3$ für $C_1$–$C_3$-Alkyl steht, B für $OR_9$ steht, $R_9$ für eine gegebenenfalls durch Halogen substituierte $C_1$–$C_3$-Alkylgruppe steht, $R_4$ $C_1$–$C_3$-Alkyl, $C_1C_3$-Alkoxy oder Halogen bedeutet, $R_5$ für $C_1$–$C_3$-Alkyl, $C_1$–$C_3$-Alkoxy oder Halogen, $R_6$ für Wasserstoff oder $C_1$–$C_3$-Alkyl stehen und $R_7$ Wasserstoff oder Methyl bedeuten; diese bevorzugte Gruppe soll als Untergruppe Ib bezeichnet werden.

Eine Gruppe besonders bevorzugter Verbindungen der Formel I sind solche, worin Ar eine substituierte Phenylgruppe bedeutet, $R_1$ $C_1$–$C_2$-Alkoxymethyl oder 2-Tetrahydrofuryl darstellt, $R_2$ Wasserstoff ist, $R_3$ für Wasserstoff oder Methyl steht, B wahlweise OH oder Methoxy bedeutet, $R_4$ Methyl ist, $R_5$ für Methyl, Chlor, $NO_2$ oder $NH_2$ steht, $R_6$ Wasserstoff, Methyl, Chlor, $NO_2$ oder $NH_2$ und $R_7$ Wasserstoff oder Methyl bedeuten. Diese besonders bevorzugte Gruppe soll als Untergruppe Ic bezeichnet werden.

Eine weitere Gruppe besonders bevorzugter Verbindungen der Formel I sind solche, worin Ar eine substituierte α-Naphthylgruppe bedeutet, $R_1$ $C_1$–$C_2$-Alkoxymethyl oder 2-Tetrahydrofuryl darstellt, $R_2$ Wasserstoff ist, $R_3$ für Wasserstoff oder Methyl steht, B wahlweise OH oder Methoxy bedeutet, $R_8$ Wasserstoff oder 3-Methyl ist, $R_{12}$ Methyl, $NO_2$ oder $NH_2$ darstellt und $R_{13}$ Wasserstoff, Methyl, $NO_2$ oder $NH_2$ bedeutet. Diese besonders bevorzugte Gruppe soll als Untergruppe Id bezeichnet werden.

Innerhalb der Untergruppe Id ist $R_{13}$ bevorzugt Wasserstoff, 4-$NO_2$ oder 4-$NH_2$.

Eine separate Gruppe von Fungiziden sind jene der Untergruppe Ic und jene der Untergruppe Id, worin $R_1$ 2-Tetrahydrofuryl bedeutet.

Besonders bevorzugt sind folgende Einzelverbindungen:

N-(2,6-Dimethylphenyl)-N-methoxyacetyl-homoserinmethylester.
N-(2,6-Dimethylphenyl)-N-methoxyacetyl-homoserinethylester.
N-(2,6-Dimethylphenyl)-N-methoxyacetyl-4-(N'-ethylcarbamoyloxy)-2-aminobuttersäuremethylester.
N-(2,6-Dimethylphenyl)-N-methoxyacetyl-[(4-imidazol-1-yl)-carbonyloxy]-buttersäuremethylester.
N-(2-Chlor-6-methoxyphenyl)-N-methoxyacetyl--homoserinmethylester.
N-(2,3,6-Trimethylphenyl)-N-methoxyacetyl-4-(N'-methylcarbamoyloxy)-2-aminobuttersäuremethylester.
N-(2,3,6-Trimethylphenyl)-N-methoxyacetyl-homoserinmethylester.
N-(2,3,6-Trimethylphenyl)-N-methoxyacetyl-homoserinethylester.
N-(2,6-Dimethyl-3-chlorphenyl)-N-methoxyacetyl-homoserinmethylester.
N-(2,3,6-Trimethylphenyl)-N-methoxyacetyl-4-methoxy-2-aminobuttersäuremethylester.
N-(2-Methylnaphthyl)-N-methoxyacetyl-homoserin
N-(2-Methylnaphthyl)-N-methoxyacetyl-4-methoxy-2-aminobuttersäuremethylester.
N-(2-Methylnaphthyl)-N-methoxyacetyl-homoserinmethylester.
N-(2-Methylnaphthyl)-N-(2-tetrahydrofurylcarbonyl)-homoserin.
N-(2-Methylnaphthyl)-N-(2-tetrahydrofurylcarbonyl)-homoserinmethylester.
N-(2-Methylnaphthyl)-N-methoxyacetyl-4-(N'-methylcarbamoyloxy)-2-aminobuttersäuremethylester.
N-(2,3-Dimethylnaphthyl)-N-methoxyacetyl-homoserinmethylester.
N-(2-Methyl-6-nitrophenyl)-N-methoxyacetyl-homoserinmethylester.

Die Verbindungen der Formel I können nach einer ganzen Reihe von Reaktionsvarianten, wie anschliessend in einem Reaktionsschema skizziert und nachfolgend im einzelnen aufgeführt, hergestellt werden. In den Formeln II bis VII haben die Reste $R_1$ bis $R_{13}$, X, Y und B die unter Formel I angegebenen Bedeutungen, Hal, Hal' und Hal'' stehen unabhängig voneinander für Halogen, vorzugsweise für Chlor, Brom oder Jod, M für ein Metallion, vorzugsweise ein Alkali- oder Erdalkaliion. Q symbolisiert eine der üblichen Abgangsgruppen, wie z.B. Halogen, insbesondere Chlor oder Brom, Benzolsulfonyl, p-Tosyl, Trifluoracetyl oder Niederalkylsulfonyl wie Mesyl.

α)

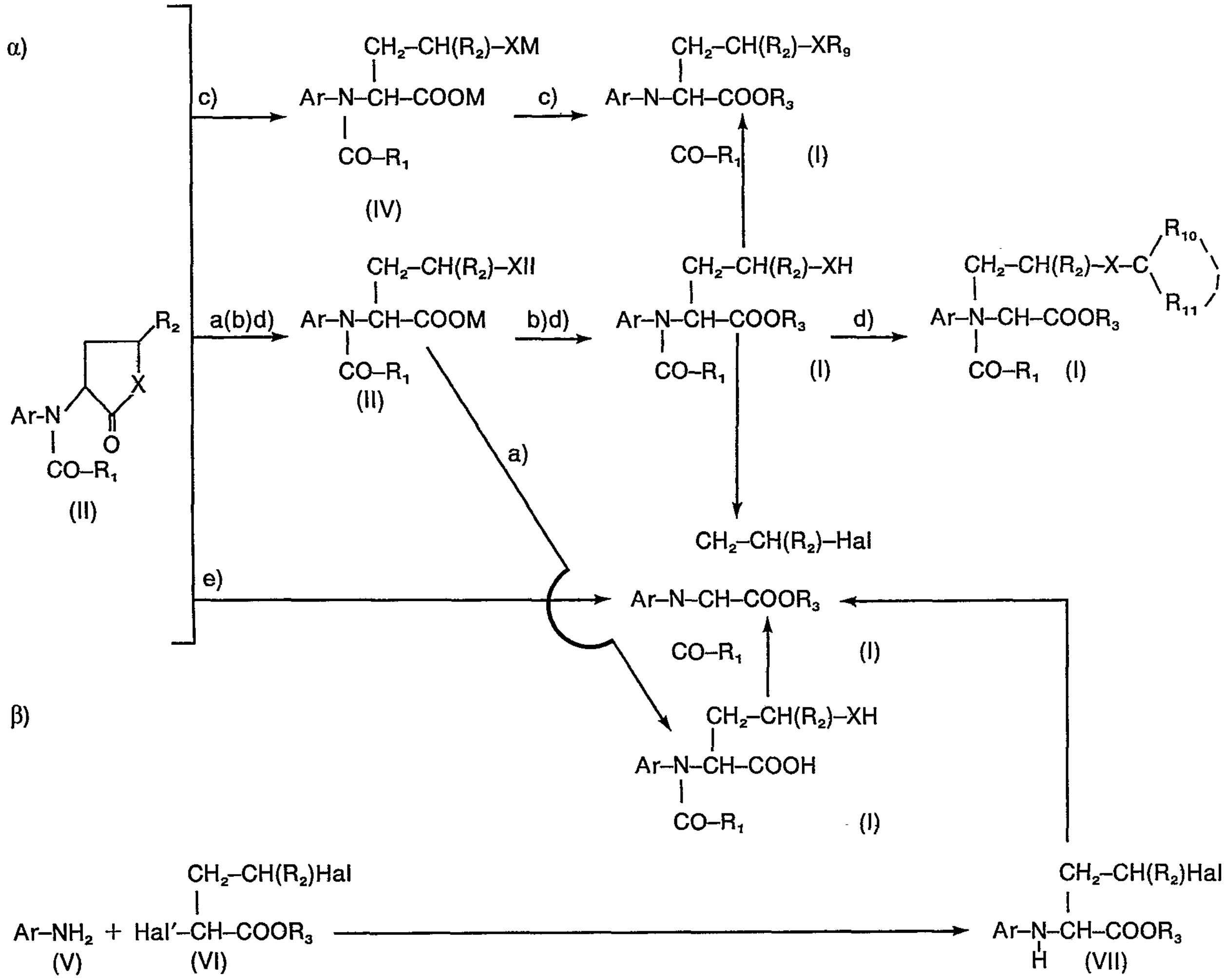


β)

(II) (IV) (I) (V) (VI) (VII)

Die Verbindungen der Formel I werden erfindungsgemäss

α) durch Ringöffnung des heterocyclischen Substituenten aus Verbindungen der Formel II hergestellt,

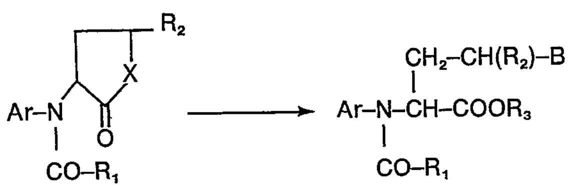

(II) (I)

a) speziell, wenn B für XH steht und $R_3$ Wasserstoff bedeutet, indem man Lacton- bzw. Thiolacton-Derivate der Formel II mit der äquimolaren Menge einer Verbindung der Formel MOH zu Carbonsäuresalzen der Formel III umsetzt

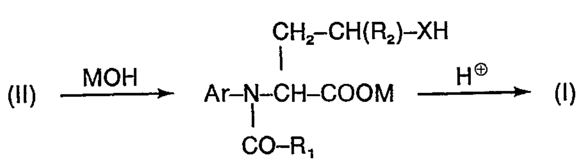


(III)

und diese durch schonende Protonierung in Produkte der Formel I überführt, oder

b) speziell, wenn B für XH steht und $R_3$ $C_1$–$C_4$-Alkyl bedeutet, indem man die Zwischenprodukte der Formel III mit Alkylierungsmitteln der Formel $R_3Q$ umsetzt

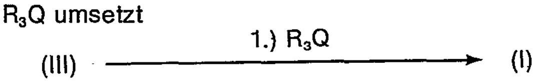


und in Produkte der Formel I überführt
oder

c) speziell wenn B für $-XR_9$ steht und $R_3$ $C_1$–$C_4$-Alkyl bedeutet, indem man die Lacton- bzw. Thiolacton-Derivate der Formel II mit 2 Äquivalenten einer Verbindung der Formel MOH zu Salzen der Formel IV umsetzt

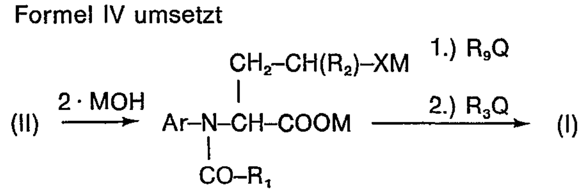


(IV)

und diese mit Verbindungen der Formeln $R_9Q$ und/oder $R_3Q$ reagieren lässt, oder indem man Produkte der Formel I, worin B für –XH steht mit Verbindungen der Formel $R_9Q$ umsetzt
oder

d) speziell, wenn B für

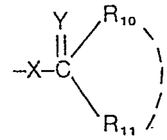

steht und $R_3$ $C_1$–$C_4$-Alkyl bedeutet, indem man die nach Variante b) gewonnenen Produkte der Formel I, worin B für –XH steht, wahlweise mit Isocyanaten bzw. Isothiocyanaten der Formel $R_{11}NCY$ oder mit Isocyan- bzw. Isothiocyansäurehalogeniden der Formel

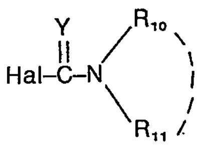

oder Harnstoff- bzw. Thioharnstoffverbindungen der Formel

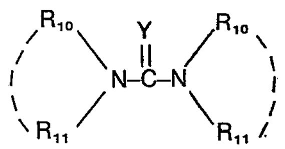

reagieren lässt,

oder

e) speziell, wenn B für Halogen steht und $R_3$ $C_1$–$C_4$-Alkyl bedeutet, indem man die Lacton- bzw. Thiolacton-Derivate der Formel II in Gegenwart eines Alkohols der Formel $R_3OH$ mit einem Halogenierungsmittel (z.B. Halogenwasserstoff, Thionylchlorid, usw.) umsetzt

$$(II). \xrightarrow{H\text{–}Hal/R_3OH} (I)$$

und, falls gewünscht, durch Reaktion mit einem Alkalihalogenid einen Halogenaustausch durchführt oder indem man bei Produkten der Formel I, worin B für –XH steht, letzteres in halogenwasserstoffsaurer Lösung in Halogen überführt,

$$B=\text{–}XH \xrightarrow[-H_2X]{+H\text{–}Hal} B=Hal$$

oder

β) durch N-Alkylierung, speziell, wenn B Halogen bedeutet, indem man ein Anilin der allgemeinen Formel V mit einer Dihalogenverbindung VI in ein Zwischenprodukt VII überführt und letzteres durch Acylierung zu Verbindungen der Formel I umsetzt

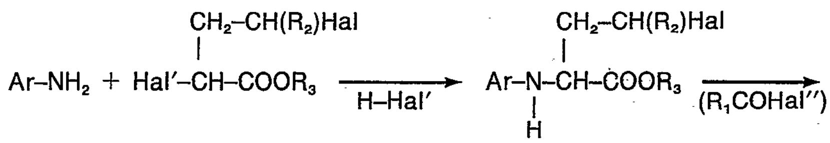


(V) (VI) (VII) (I).

Bei allen Herstellungsvarianten ist es vorteilhaft, Lösungsmittel zu verwenden, die sich gegenüber allen Reaktionsteilnehmern inert verhalten, und es ist zweckmässig, zur Beschleunigung der Reaktionsgeschwindigkeit die Reaktionstemperatur zu erhöhen und/oder geeignete Katalysatoren zuzusetzen. In manchen Fällen ist es zweckmässig, ein Kondensations- oder Bindemittel dem Reaktionsgemisch beizugeben. In einigen Fällen kann es zweckmässig erscheinen, einzelne Reaktionsschritte unter Schutzgasatmosphäre durchzuführen.

Bei den verschiedenen Herstellungsvarianten können folgende Bedingungen von Vorteil sein:

Die alkalische Ringöffnung der Ausgangsverbindung II erfolgt bei der Herstellungsvariante a) zweckmässigerweise in stark polaren Lösungsmitteln, vorzugsweise Alkohol-Wasser-Gemischen wie z.B. wasserhaltigem Methanol. Als Hydroxide werden hierbei vorzugsweise Alkali- oder Erdalkalihydroxide verwendet, insbesondere Natriumhydroxid. Die Reaktionstemperatur kann bei dieser Umsetzung in einem Bereich von $-10°$ bis $+100$ °C liegen. Die anschliessende Protonierung [III → I] wird vorteilhafterweise unter schonenden Bedingungen, vorzugsweise mit Hilfe von sauren Ionenaustauscherharzen durchgeführt.

Die als Herstellungsvariante b) definierte Veresterung der COOH-Gruppe in Verbindungen der Formel III mit Verbindungen der Formel $R_3Q$ durchgeführt, wobei $R_3$ wie unter Formel I definiert ist und Q für eine der üblichen Abgangsgruppen eines Alkylierungsmittels steht, wie z.B. Halogen, insbesondere Chlor oder Brom, Benzolsulfonyl, p-Tosyl, Trifluoracetyl oder Niederalkyl wie Mesyl. Hierbei werden vorteilhafterweise dipolare aprotische Lösungsmittel verwendet, wie z.B. Dimethylformamid, Dimethylsulfoxid oder Hexamethylphosphorsäuretriamid, aber auch andere Kohlenwasserstoffe, insbesondere halogenierte Kohlenwasserstoffe, die gegenüber der Verbindung III inert sind, kommen als Reaktionsmedium in Frage. Diese Umsetzung kann in einem Temperaturbereich von 0° bis $+100$ °C, vorzugsweise $+10°$ bis $+40$ °C, erfolgen.

Für die Herstellungsvariante c) werden als Lösungs- und Reaktionsmittel zweckmässigerweise wässrige Lösungen der Alkohole $R_9OH$ und/oder $R_3OH$ verwendet, wobei $R_9$ und $R_3$ identisch sein können und die gleichen Bedeutungen wie unter Formel I haben. Als Hydroxide kommen hierbei hauptsächlich Alkali- oder Erdalkalihydroxide, insbesondere NaOH in Frage. Die Reaktionstemperatur kann bei dieser Umsetzung in einem Bereich von $-10°$ bis $+100$ °C liegen.

Variante d), die sich besonders zur Herstellung von Verbindungen der Formel I mit

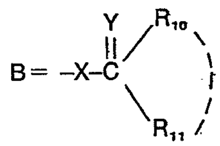

eignet, wird vorzugsweise in inerten aprotischen Lösungsmitteln durchgeführt. Als Lösungsmittel eignen sich halogenierte Kohlenwasserstoffe wie z. B. Dichlormethan, Chloroform, Tetrachlorkohlenstoff, 1,2-Dichloräthan, Tetrachloräthylen, Chlorbenzol usw., aber auch aromatische Kohlenwasserstoffe wie Benzol, Toluol, Xylole oder Nitrile wie Acetonitril, Propionitril und Ester wie Ethylacetat, Butylacetat usw. Auch Gemische solcher Lösungsmittel können verwendet werden. Die Temperatur kann bei dieser Umsetzung in einem Bereich von 0° bis 80 °C liegen, vorzugsweise 0° bis 30 °C. In manchen Fällen ist es zweckmässig, einen Katalysator zuzusetzen; es eignen sich als Katalysatoren z. B. tertiäre Amine wie Trialkylamine (Trimethylamin, Triethylamin, Tripropylamin) oder auch Diazabicyclo-(2,2,2)octan. Insbesondere bei dieser Variante kann ein Arbeiten unter Schutzgasatmosphäre, z. B. unter Stickstoff, gewisse Vorteile haben.

Bei der Variante e) wird die Umsetzung der Ausgangsverbindung II vorzugsweise in Alkoholen der Formel $R_3OH$, worin $R_3$ = $C_1$–$C_4$-Alkyl bedeutet, durchgeführt. Ein Zusatz eines weiteren inerten Lösungsmittels ist möglich. Die Reaktionstemperatur richtet sich bei dieser Herstellungsvariante nach der Art des eingesetzten Halogenierungsmittels. Wird z. B. Halogenwasserstoff verwendet, so kann die Temperatur in einem Bereich von −20° bis 120 °C, vorzugsweise jedoch bei 0° bis 80 °C liegen. Wird als Halogenierungsmittel aber ein Thionylhalogenid eingesetzt, so liegt die Temperatur im allgemeinen in einem Bereich von −20° bis +30 °C. Halogen bedeutet bei dieser Variante Fluor, Chlor, Brom und Jod, vorzugsweise Chlor und Jod, dabei sind Jodide auch prinzipiell durch Umhalogenierung z. B. mit Kaliumjodid aus den entsprechenden Chloriden zugänglich.

Die N-Alkylierung β) erfolgt zweckmässigerweise in einem der üblichen inerten organischen Lösungsmittel wie z. B. Benzol, Toluol, Xylole, Tetrachlorkohlenstoff, Tetrachlorethylen, Diethylether, t-Butylmethylether, Tetrahydrofuran usw. Es kann sich als vorteilhaft erweisen, die Reaktion in Gegenwart eines Protonenacceptors wie z. B. $NaHCO_3$ oder $Na_2CO_3$ durchzuführen. Als Acylierungsmittel eignen sich z. B. Verbindungen der Formeln $R_1COHal''$ oder $(R_1CO)_2O$, worin $R_1$ die unter Formel I aufgeführten Bedeutungen hat.

Alle Verbindungen der Formel I, bei denen X für ein Schwefelatom steht, lassen sich mit Oxidationsmitteln wie z. B. Persäuren wie $H_2O_2$, Perbenzoesäure, Metachlorperbenzoesäure, $HJO_4$ oder auch mit Kaliumpermanganat nachoxidieren. Dabei lässt sich eine HS-Gruppe in eine $HO_3S$-Gruppe überführen, ein Thioether in ein Sulfoxid bzw. weiter zu einem Sulfon.

Das Herstellungsverfahren ist in allen seinen Varianten α[a, b, c, d, e] und β ein wesentlicher Bestandteil der Erfindung.

Die Verbindungen der Formel I besitzen in Nachbarstellung zum Stickstoffatom ein (*) und im Falle von $R_2$ = $CH_3$, benachbart zu $R_2$ ein zweites (**) Asymmetriezentrum

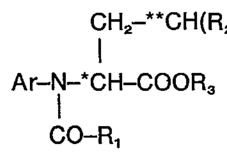
(I)

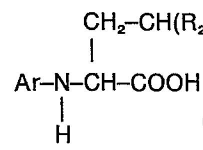
(VIII)

und können auf die übliche Art in optische Isomere bzw. Diastereomere gespalten werden; so z. B. durch fraktionierte Kristallisation oder chromatographische Trennung eines Salzes von VIII mit einer optisch aktiven Base (z. B. D-α-Phenyläthylamin) und anschliessender Acylierung der optisch aktiven Verbindungen VIII zu I. Die optischen Isomeren bzw. Diastereomeren I besitzen unterschiedliche mikrobizide Wirkungen.

Je nach Substitution können auch weitere asymmetrische Kohlenstoffatomen im Molekül vorhanden sein.

Unabhängig von der genannten optischen Isomerie wird eine Atropisomerie um die >N-Ar-Achse beobachtet, wenn Ar unsymmetrisch zu dieser Achse substituiert ist.

Sofern keine gezielte Synthese zur Isolierung reiner Isomerer durchgeführt wird, fällt normalerweise ein Produkt der Formel I als ein Gemisch aller dieser möglichen Isomeren an.

Die Ausgangsverbindungen der Formeln V, VI und VII sind allgemein bekannt und werden nach allgemein bekannten Verfahren hergestellt.

Verbindungen, die von der allgemeinen Formel II umfasst werden, sind grossenteils aus DE-OS 2 804 299 (= GB 1 577 702) bekannt. Nichtbekannte Einzelverbindungen der Formel II lassen sich nach einem der dort genannten Herstellungsverfahren gewinnen.

Einige Lacton-Derivate der Formel II werden auch in der DE-OS 2 724 786 genannt. Lacton- und Thiolactonderivate der Formel II werden als Fungizide erwähnt in DE-OS 2 845 454.

Die Zwischenprodukte der Formeln III und IV sind neu und wirken auch fungizid. Aus dem Stand der Technik sind bereits mikrobizide N-Acyl-N-aryl-α-aminoalkancarbonsäureester bekanntgeworden. So werden in der DE-3010412 als strukturnächste Verbindungen N-Acyl-N-aryl-α-aminobuttersäureester mit Wirkung als Pflanzenfungizide vorbeschrieben. Präparate gleichen Typs werden zum selben Zwecke auch in der EP-PS-17850 vorgeschlagen. N-Acyl-N-aryl-α-aminopropionsäure-Derivate, die man auch als N-Acyl-N-aryl-alanine auffassen kann, werden sowohl in der DE-2513732 wie auch in der DE-2515091 als Mikrobizide, besonders zur Bekämpfung von Pflanzenkrankheiten beschrieben. Gegenüber diesen nächstvergleichbaren Verbindungen des Standes der Technik und älterer Anmeldungen zeigen die Homoserin-Verbindungen der Formel I vorliegender Erfin-

dung eine ganz unerwartete und wesentlich höhere Aktivität gegen Pflanzenkrankheiten.

Die nachfolgenden Beispiele sollen den Erfindungsgegenstand erläutern, ohne ihn einzuschränken Prozent- und Teile-Angaben beziehen sich stets auf Gewicht. Temperaturen sind in Celsiusgraden angegeben. Sofern nicht speziell vermerkt, ist bei der Nennung eines Wirkstoffes der Formel I stets das racemische Gemisch gemeint.

Herstellungsbeispiele:

Beispiel 1:

Herstellung von

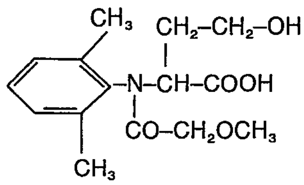

(1.1)

N-(2,6-Dimethylphenyl)-N-methoxyacetyl-homoserin:

11,0 g (0,04 Mol) 3-[N-(Methoxyacetyl)-N-(2,6-dimethylphenyl)]-amino-tetrahydro-2-furanon werden in 50 ml Methanol gelöst und mit einer Lösung von 1,6 g Natriumhydroxid in 20 ml Wasser versetzt. Man rührt 12 Stunden bei Raumtemperatur, dampft die Lösung ein und protoniert das erhaltene Salz mit einer sauren Ionenaustauscher-Säule. Das wässrige Eluat wird mit Methylenchlorid extrahiert, die vereinigten Extrakte mit Wasser gewaschen, über Natriumsulfat getrocknet und eingedampft. Der Rückstand wird aus Essigsäureethylester/Ligroin umkristallisiert. Man erhält farblose Kristalle vom Smp. 150–152°.

Auf analoge Weise lassen sich weitere Verbindungen der Formel I, insbesonders die der nachfolgenden Untergruppe Ia herstellen:

Tabelle 1
($R_2$ = H; $R_3$ = H;)

| Verb. Nr. | Ar | B | $R_1$ | Physik. Konstante |
|---|---|---|---|---|
| 1.1 | $C_6H_3(CH_3)_2(2,6)$ | OH | $CH_2OCH_3$ | Smp. 150–152°C |
| 1.2 | $C_6H_2(CH_3)_3(2,3,6)$ | OH | $CH_2OCH_3$ | Smp. 155–157°C |
| 1.3 | $C_6H_3(CH_3)_2(2,6)$ | SH | $CH_2OCH_3$ | |
| 1.4 | $C_6H_2(CH_3)_2(2,6)Cl(3)$ | OH | $CH_2OCH_3$ | |
| 1.5 | $C_6H_3(CH_3)_2(2,6)$ | OH | 2-Tetrahydrofuryl | Smp. 166–170°C |
| 1.6 | α-Naphthyl-$CH_3(2)$ | OH | $CH_2OC_2H_5$ | |
| 1.7 | $C_6H_3(CH_3)_2(2,6)$ | OH | Cyclopropyl | |
| 1.8 | $C_6H_3(CH_3)_2(2,6)$ | OH | $CH=CH-CH_3$ | |
| 1.9 | $C_6H_3(CH_3)_2(2,6)$ | OH | $CH_2OC_2H_5$ | |
| 1.10 | $C_6H_3CH_3(2)C_2H_5(6)$ | OH | $CH_2OCH_3$ | |
| 1.11 | α-Naphthyl-$CH_3(2)$ | SH | $CH_2OCH_3$ | |
| 1.12 | α-Naphthyl-$CH_3(2)$ | OH | $CH_2OC_2H_5$ | |
| 1.13 | $C_6H(CH_3)_4(2,3,5,6)$ | OH | 2-Furyl | |
| 1.14 | $C_6H_2(CH_3)_3(2,3,6)$ | OH | 2-Furyl | |
| 1.15 | $C_6H_3(CH_3)(2)NO_2(6)$ | OH | $CH_2OCH_3$ | Smp. 110–114°C |
| 1.16 | $C_6H_2(CH_3)_3(2,3,6)$ | SH | Cyclopropyl | |
| 1.17 | α-Naphthyl$(CH_3)_2(2,3)$ | OH | $CH_2OC_2H_5$ | |
| 1.18 | $C_6H_3(CH_3)_2(2,6)$ | OH | $CH_2OCH_3$ | |
| 1.19 | $C_6H_3(CH_3)_2(2,6)$ | SH | $CH_2OCH_3$ | |
| 1.20 | $C_6H_2(CH_3)_3(2,3,6)$ | OH | $CH_2OC_2H_5$ | |
| 1.24 | α-Naphthyl-$(CH_3)_2(2,3)$ | OH | $CH_2OCH_3$ | Smp. 160–161°C |
| 1.25 | α-Naphthyl-$CH_3(2)$ | OH | $CH_2OCH_3$ | Smp. 125–134°C |
| 1.26 | α-Naphthyl-$CH_3(2)$ | OH | 2-Tetrahydrofuryl | |
| 1.27 | α-Naphthyl-$CH_3(2)$-$NO_2(4)$ | OH | 2-Tetrahydrofuryl | |
| 1.28 | α-Naphthyl-$CH_3(2)$-$NH_2(4)$ | OH | 2-Tetrahydrofuryl | |
| 1.29 | α-Naphthyl-$(CH_3)_2(2,3)$-$NO_2(4)$ | OH | 2-Tetrahydrofuryl | |
| 1.30 | α-Naphthyl-$(CH_3)_2(2,3)$-$NH_2(4)$ | OH | 2-Tetrahydrofuryl | |
| 1.31 | α-Naphthyl-$(CH_3)_2(2,3)$-$NO_2(4)$ | OH | $CH_2OCH_3$ | |
| 1.32 | α-Naphthyl-$(CH_3)_2(2,3)$-$NH_2(4)$ | OH | $CH_2OCH_3$ | |
| 1.33 | α-Naphthyl-$(CH_3)(2)$-$NO_2(4)$ | OH | $CH_2OCH_3$ | |
| 1.34 | α-Naphthyl-$(CH_3)(2)$-$NO_2(6)$ | OH | $CH_2OCH_3$ | |

Beispiel 2a:

Herstellung von

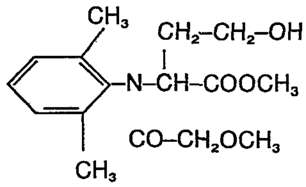 (2.1)

N-(2,6-Dimethylphenyl)-N-methoxyacetylhomoserinmethylester:

11,0 g (0,04 Mol)
3-[N-Methoxyacetyl)-N-(2,6-dimethylphenyl)-aminotetrahydro-2-furanon
werden in 50 ml Methanol gelöst, mit einer Lösung von 1,6 g Natriumhydroxid in 20 ml Wasser versetzt und 12 Stunden gerührt, eingedampft und der Rückstand in 75 ml absolutem Dimethylformamid gelöst. Man gibt nun tropfenweise 3,2 ml Methyljodid zu, rührt 24 Stunden bei Raumtemperatur und lässt weitere 1,8 ml Methyljodid zutropfen. Nach 24 stündigem Rühren bei Raumtemperatur wird das Lösungsmittel im Vakuum entfernt, der Rückstand in Methylenchlorid aufgenommen, mit Wasser gewaschen, die Lösung über Natriumsulfat getrocknet und eingedampft. Der trockene Rückstand wird in Ether aufgenommen, mit Petrolether ausgefällt, filtriert und zweimal mit Ether/Petrolether digeriert. Die erhaltene Kristalle schmelzen bei 81–83°.

Beispiel 2b:

Herstellung von

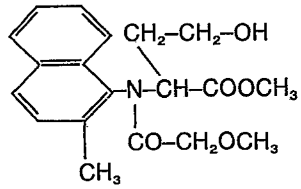 (verb. Nr. 2.19)

N-(2-Methylnaphthyl)-N-methoxyacetyl-homoserinmethylester

α) 15,7 g
N-(2-Methylnaphthyl)-N-methoxyacetyl-N-(2-oxo-tetrahydrofuran-3-yl)-amin
werden in 100 ml Methanol gelöst. Bei 0 °C lässt man 10 ml Natronlauge (30%ig) während 10 Minuten zutropfen und rührt anschliessend 3 Stunden bei Raumtemperatur. Danach engt man die Lösung am Rotationsverdampfer ein und trocknet das verbliebene Dinatriumsalz des N-(2-Methylnaphthyl)-N-methoxyacetyl-homoserins bei 90 °C am Hochvakuum.

β) 0,083 Mole des erhaltenen Dinatriumsalzes werden in 100 ml Dimethylformamid gelöst und bei 0–5° während 15 Minuten mit 14,1 g Methyljodid versetzt. Man rührt 6 Stunden bei Raumtemperatur, engt die Lösung am Rotationsverdampfer ein, verrührt den Rückstand mit 50 ml Methylenchlorid, giesst die organische Phase auf Eiswasser, trennt ab, schüttelt noch zweimal mit je 25 ml Methylenchlorid aus und trocknet die vereinigten Extrakte über Na-Sulfat. Nach dem Einengen der Lösung löst man den Rückstand in 30 ml heissem Chloroform und gibt nach dem Abkühlen wenig Diäthyläther zu: 14 g kristallines Endprodukt, Smp. 132–137 °C.

Auf analoge Weise lassen sich weitere Verbindungen der Formel I herstellen.

Tabelle 2
($R_2$ = H)

| Verb. Nr. | Ar | B | $R_1$ | $R_3$ | Physik. Konst. |
|---|---|---|---|---|---|
| 2.1 | $C_6H_3(CH_3)_2(2,6)$ | OH | $CH_2OCH_3$ | $CH_3$ | Smp. 81–83° |
| 2.2 | $C_6H_3(CH_3)_2(2,6)$ | OH | $CH_2OCH_3$ | $C_2H_5$ | Smp. 87–90° |
| 2.3 | $C_6H_2(CH_3)_3(2,3,6)$ | OH | $CH_2OCH_3$ | $CH_3$ | Harz |
| 2.4 | $C_6H_2(CH_3)_2(2,6)Cl(3)$ | OH | $CH_2OCH_3$ | $CH_3$ | Harz |
| 2.5 | $C_6H_2(CH_3)_3(2,3,6)$ | OH | $CH_2OCH_3$ | $C_2H_5$ | Smp. 98–102° |
| 2.6 | $C_6H_3(CH_3)_2(2,6)$ | SH | $CH_2OCH_3$ | $CH_3$ | Smp. 118–121° |
| 2.7 | $C_6H(CH_3)_4(2,3,5,6)$ | OH | $CH_2OCH_3$ | $CH_3$ | |
| 2.8 | $C_6H_3(CH_3)_2(2,6)$ | OH | $CH_2OCH_3$ | $C_4H_9$–n | |
| 2.9 | $C_6H_3(CH_3)_2(2,6)$ | OH | $CH_2OCH_3$ | $C_3H_7$–i | |
| 2.10 | $C_6H_3OCH_3(2)Cl(6)$ | OH | $CH_2OCH_3$ | $CH_3$ | Harz |
| 2.11 | $C_6H_3OCH_3(2)CH_3(6)$ | OH | $CH_2OCH_3$ | $CH_3$ | |
| 2.12 | $C_6H_3(CH_3)_2(2,6)$ | OH | 2-Furyl | $CH_3$ | |
| 2.13 | $C_6H_3(CH_3)_2(2,6)$ | OH | 2-Tetrahydro-furyl | $CH_3$ | Smp. 104–110° |
| 2.14 | $C_6H_3(CH_3)_2(2,6)$ | OH | Cyclopropyl | $CH_3$ | Harz |
| 2.15 | $C_6H_2(CH_3)_3(2,3,6)$ | OH | $CH_2OC_2H_5$ | $CH_3$ | |
| 2.16 | $C_6H_2(CH_3)_2(2,6)Br(4)$ | SH | $CH=CH-CH_3$ | $C_2H_5$ | |
| 2.17 | $C_6H_3(CH_3)_2(2,6)$ | OH | 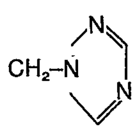 | $CH_3$ | |

Tabelle 2 (Fortsetzung)

| Verb. Nr. | Ar | B | $R_1$ | $R_3$ | Physik. Konst. |
|---|---|---|---|---|---|
| 2.18 | $C_6H_2(CH_3)_3(2,3,6)$ | OH | 2-Tetrahydro-furyl | | |
| 2.19 | α-Naphthyl-$CH_3(2)$ | OH | $CH_2OCH_3$ | $CH_3$ | Smp. 132–137° |
| 2.20 | α-Naphthyl$(CH_3)_2(2,3)$ | SH | $CH_2OCH_3$ | $CH_3$ | |
| 2.21 | $C_6H_3(CH_3)_2(2,6)$ | OH | $CH_2OCH_3$ | 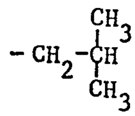 | |
| 2.22 | α-Nahpthyl-$CH_3(2)OCH_3(3)$ | OH | $CH_2OCH_3$ | $CH_3$ | |
| 2.23 | α-Naphthyl-$CH_3(2)Cl(3)$ | OH | $CH_2OCH_3$ | $CH_3$ | |
| 2.24 | α-Naphthyl-$CH_3(2)$ | OH | 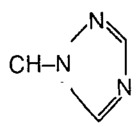 | $CH_3$ | |
| 2.25 | $C_6H_2(CH_3)_2(2,6)OCH_3(3)$ | OH | $CH_2OC_2H_5$ | $CH_3$ | |
| 2.26 | $C_6H_3(CH_3)_{32}(2,6)$ | OH | $CH_2OCH_3$ | $CH_3$ | |
| 2.27 | $C_6H_2(CH_3)_3(2,3,6)$ | OH | 2-Tetrahydro-furyl | $CH_3$ | Harz |
| 2.31 | $C_6H_3CH_3(2)C_2H_5(6)$ | OH | 2-Tetrahydro-furyl | $CH_3$ | Harz |
| 2.32 | $C_6H_3CH_3(2)NO_2(6)$ | OH | $CH_2OCH_3$ | $CH_3$ | Harz |
| 2.33 | α-Naphthyl$(CH_3)_2(2,3)$ | OH | $CH_2OCH_3$ | $CH_3$ | Smp. 137° |
| 2.34 | α-Naphthyl$(CH_3)_2(2,3)$ | OH | $CH_2OCH_3$ | $C_2H_5$ | Smp. 115° |
| 2.35 | α-Naphthyl$(CH_3)_2(2,3)NO_2(4)$ | OH | $CH_2OCH_3$ | $CH_3$ | |
| 2.36 | α-Naphthyl$(CH_3)_2(2,3)NH_2(4)$ | OH | $CH_2OCH_3$ | $CH_3$ | |
| 2.37 | α-Naphthyl$(CH_3)_2(2,3)NO_2(4)$ | OH | $CH_2OCH_3$ | $C_2H_5$ | |
| 2.38 | α-Naphthyl$(CH_3)_2(2,3)NO_2(4)$ | OH | 2-Tetrahydro-furyl | $CH_3$ | |
| 2.39 | α-Naphthyl$(CH_3)_2(2,3)NO_2(4)$ | OH | 2-Tetrahydro-furyl | $C_2H_5$ | |
| 2.40 | α-Naphthyl$(CH_3)_2(2,3)NH_2(4)$ | OH | 2-Tetrahydro-furyl | $CH_3$ | |
| 2.41 | α-Naphthyl$(CH_3)_2(2,3)NO_2(6)$ | OH | $CH_2OCH_3$ | $CH_3$ | |
| 2.42 | α-Naphthyl-$CH_3(2)$ | OH | 2-Tetrahydro-furyl | $CH_3$ | Smp. 141–145° |
| 2.43 | α-Naphthyl-$CH_3(2)$ | OH | 2-Tetrahydro-furyl | $C_2H_5$ | |
| 2.44 | α-Naphthyl-$CH_3(2)NO_2(4)$ | OH | 2-Tetrahydro-furyl | $CH_3$ | |
| 2.45 | α-Naphthyl-$CH_3(2)NO_2(4)$ | OH | $CH_2OCH_3$ | $CH_3$ | |
| 2.46 | α-Naphthyl-$CH_3(2)NH_2(4)$ | OH | $CH_2OCH_3$ | $CH_3$ | |
| 2.47 | α-Naphthyl-$CH_3(2)NO_2(4)$ | OH | $CH_3OCH_3$ | $C_2H_5$ | |
| 2.48 | α-Naphthyl-$CH_3(2)NH_2(4)$ | OH | 2-Tetrahydro-furyl | $CH_3$ | |

Beispiel 3:

Herstellung von

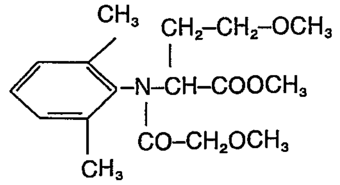

(3.1)

N-(2,6-Dimethylphenyl)-N-methoxyacetyl-4-methoxy-2-amino-buttersäuremethylester:

13,8 g 3-[(N-methoxyacetyl)-N-(2,6-dimethylphenyl)]-amino-tetrahydro-2-furanon in 100 ml Methanol werden mit 4,0 g Natriumhydroxid in 20 ml Wasser versetzt, 1 Stunde bei Raumtemperatur stehengelassen und dann eingedampft. Der Rückstand wird im Vakuum getrocknet, in 100 ml Dimethylformamid aufgenommen und unter Rühren tropfenweise mit 14 ml Methyljodid versetzt, hierbei steigt die Temperatur auf ca 35° an, und es fällt ein Niederschlag aus. Man rührt noch 12 Stunden bei Raumtemperatur, dampft das Lösungsmittel ein und versetzt den Rückstand mit Wasser. Nach mehrmaliger Extraktion mit Methylenchlorid werden die vereinigten Extrakte wiederholt mit

Wasser gewaschen, über Natriumsulfat getrocknet und eingedampft. Die Destillation bei 164–168°/0,8 Torr führt zu einem zähen Öl.

Auf analoge Weise lassen sich weitere Verbindungen der Formel I, insbesondere die der nachfolgenden Untergruppe Ic herstellen:

Tabelle 3
($R_2$ = H; B = $XR_9$)

| Verb. Nr. | Ar | –X– | $R_1$ | $R_3$ | $R_9$ | Physik. Konst. |
|---|---|---|---|---|---|---|
| 3.1 | $C_6H_3(CH_3)_2(2,6)$ | O | $CH_2OCH_3$ | $CH_3$ | $CH_3$ | Sdp. 164–168°/ 0,8 Torr |
| 3.2 | $C_6H_3(CH_3)_2(2,6)$ | O | $CH_2OCH_3$ | $C_2H_5$ | $C_2H_5$ | Sdp. 195°/ 0,8 Torr |
| 3.3 | $C_6H_3(CH_3)_2(2,6)$ | S | $CH_2OCH_3$ | $CH_3$ | $CH_3$ | Smp. 51–52° |
| 3.4 | $C_6H_2(CH_3)_3(2,3,6)$ | O | $CH_2OCH_3$ | $CH_3$ | $CH_3$ | Harz |
| 3.5 | $C_6H_3(CH_3)_2(2,6)$ | O | 2-Tetrahydrofuryl | $CH_3$ | $CH_3$ | |
| 3.6 | $C_6H_2(CH_3)_2(2,6)Cl(3)$ | O | $CH_2OCH_3$ | $CH_3$ | $CH_3$ | |
| 3.7 | $C_6H_3CH_3(2)C_2H_5(6)$ | O | $CH_2OCH_3$ | $C_2H_5$ | $C_2H_5$ | |
| 3.8 | $C_6H(CH_3)_4(2,3,5,6)$ | O | 2-Furyl | $CH_3$ | $CH_3$ | |
| 3.9 | α-Naphthyl-$CH_3$(2) | O | 2-Tetrahydrofuryl | $CH_3$ | $CH_3$ | |
| 3.10 | α-Naphthyl-$CH_3$(2) | O | $CH_2OCH_3$ | $CH_3$ | $CH_3$ | Harz |
| 3.11 | $C_6H_3Cl(2)OCH_3(6)$ | O | $CH_2OCH_3$ | $C_3H_7$–n | $C_3H_7$–n | |
| 3.12 | $C_6H_3Cl(2)OCH_3(6)$ | O | 2-Tetrahydrofuryl | $CH_3$ | $CH_3$ | |
| 3.13 | α-Naphthyl-$CH_3$(2) | S | 2-Furyl | $CH_3$ | $CH_3$ | |
| 3.14 | $C_6H_3(CH_3)_2(2,6)$ | O | Cyclopropyl | $C_2H_5$ | $CH_3$ | |
| 3.15 | $C_6H_3(CH_3)_2(2,6)$ | O | $CH_2OCH_3$ | $CH_3$ | $C_2H_5$ | |
| 3.16 | $C_6H_3(CH_3)_2(2,6)$ | O | 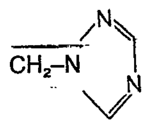 | $CH_3$ | $CH_3$ | |
| 3.17 | $C_6H_3(CH_3)_2(2,6)$ | O | $CH_2OCH_3$ | $CH_3$ | $CH_3$ | |
| 3.18 | $C_6H_3(CH_3)_2(2,6)$ | O | $CH_2OCH_3$ | $C_2H_5$ | $C_2H_5$ | |
| 3.19 | $C_6H_3(CH_3)_2(2,6)$ | O | 2-Tetrahydrofuryl | $CH_3$ | $CH_3$ | |
| 3.24 | α-Naphthyl-$CH_3$(2)-$NO_2$(4) | O | $CH_2OCH_3$ | $CH_3$ | $CH_3$ | |
| 3.25 | α-Naphthyl-$CH_3$(2)-$NO_2$(4) | O | 2-Tetrahydrofuryl | $CH_3$ | $CH_3$ | |
| 3.26 | α-Naphthyl-$CH_3$(2)-$NH_2$(4) | O | 2-Tetrahydrofuryl | $CH_3$ | $CH_3$ | |
| 3.27 | α-Naphthyl-$CH_3$(2)-$NH_2$(4) | O | $CH_2OCH_3$ | $CH_3$ | $CH_3$ | |
| 3.28 | α-Naphthyl$(CH_3)_2$-(2,3)$NO_2$(4) | O | $CH_2OCH_3$ | $CH_3$ | $CH_3$ | |
| 3.29 | α-Naphthyl$(CH_3)_2$(2,3)-$NO_2$(4) | O | 2-Tetrahydrofuryl | $CH_3$ | $CH_3$ | |
| 3.30 | α-Naphthyl$(CH_3)_2$(2,3)-$NH_2$(4) | O | 2-Tetrahydrofuryl | $CH_3$ | $CH_3$ | |
| 3.31 | α-Naphthyl$(CH_3)_2$(2,3)-$NH_2$(4) | O | $CH_2OCH_3$ | $CH_3$ | $CH_3$ | |

Beispiel 4:

a) Herstellung von

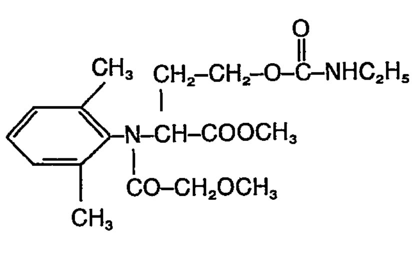

N-(2,6-Dimethylphenyl)-N-methoxyacetyl-4-(N-ethylcarbamoyloxy)-2-aminobuttersäure-methylester:

9,3 g N-(2,6-Dimethylphenyl)-N-methoxyacetyl-homoserinmethylester werden in 200 ml absolutem Tetrahydrofuran gelöst und mit einer katalytischen Menge 1,4-Diazabicyclo(2,2,2)octan versetzt. Man lässt unter Eiskühlung und Rühren 2,6 g Ethylisocyanat zutropfen, rührt 20 Stunden bei einer Temperatur von 40–50°, dampft die Lösung ein und digeriert das verbleibende Harz mit Petrolether, wobei dieses erstarrt. Man filtriert die erhaltene Kristalle ab; ihr Schmelzpunkt beträgt 56–60°.

b) Herstellung von

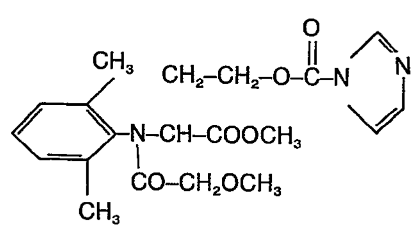

(4.2)

N-(2,6-Dimethylphenyl)-N-methoxyacetyl-[4-imidazol-1-yl)-carbonyloxy]-buttersäuremethylester:

9,3 g N-(2,6-Dimethylphenyl)-N-methoxyacetylhomoserinmethylester

werden in 200 ml absolutem Dioxan gelöst und unter Stickstoffatmosphäre mit 7,3 g N,N-Carbonyldiimidazol versetzt. Die resultierende Lösung wird über Nacht bei Raumtemperatur gerührt, auf Eiswasser gegossen und mit Methylenchlorid extrahiert. Die Extrakte werden mit Wasser gewaschen, über Natriumsulfat getrocknet und das Lösungsmittel im Vakuum entfernt. Nach Lösen in Ether und Behandeln mit Aktivkohle resultiert das reine Produkt in Form eines viskosen Harzes.

Analog lassen sich weitere Verbindungen der Formel I herstellen.

Tabelle 4

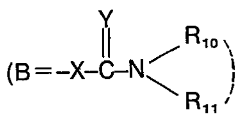 ; $R_2$=H)

| Verb. Nr. | Ar | $R_1$ | $R_3$ | B | Physik. Konst. |
|---|---|---|---|---|---|
| 4.1 | $C_6H_3(CH_3)_2$(2,6) | $CH_2OCH_3$ | $CH_3$ | $-OCO-NHC_4H_9$-n | Öl |
| 4.2 | $C_6H_3(CH_3)_2$(2,6) | $CH_2OCH_3$ | $CH_3$ | -OCO-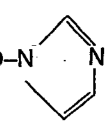 | Harz |
| 4.3 | $C_6H_3(CH_3)_2$(2,6) | $CH_2OCH_3$ | $CH_3$ | $-OCO-NHC_2H_5$ | Smp. 56–60° |
| 4.4 | $C_6H_3(CH_3)_2$(2,6) | $CH_2OCH_3$ | $CH_3$ | $-OCO-NHCH_3$ | Smp. 99–104° |
| 4.5 | $C_6H_2(CH_3)_3$(2,3,6) | $CH_2OCH_3$ | $CH_3$ | $-OCO-NH-CH(CH_3)_2$ | Harz |
| 4.6 | $C_6H_3(CH_3)_2$(2,6) | $CH_2OCH_3$ | $CH_3$ | $-OCO-NH-C_6H_3Cl_2$(3,5) | Smp. 129–130° |
| 4.7 | $C_6H_2(CH_3)_2$(2,6)Cl(3) | $CH_2OCH_3$ | $CH_3$ | $-OCO-NHC_2H_5$ | Harz |
| 4.8 | $C_6H(CH_3)_4$(2,3,5,6) | $CH_2OCH_3$ | $CH_3$ | -OCO-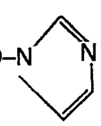 | |
| 4.9 | $C_6H_2(CH_3)_3$(2,3,6) | $CH_2OCH_3$ | $CH_3$ | $-OCO-NHCH_3$ | Harz |
| 4.10 | $C_6H_3CH_3$(2)Cl(6) | $CH_2OCH_3$ | $CH_3$ | $-OCO-NHC_3H_7$-n | |
| 4.11 | $C_6H_3CH_3(2)OCH_3$(6) | $CH_2OCH_3$ | $CH_3$ | -OCO-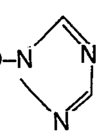 | |
| 4.12 | $C_6H_3OCH_3$(2)Cl(6) | $CH_2OCH_3$ | $CH_3$ | -OCO-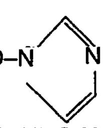 | |
| 4.13 | $C_6H_3(CH_3)_2$(2,6) | $CH_2OCH_3$ | $C_2H_5$ | $-OCO-NHC_2H_5$ | |
| 4.14 | α-Naphthyl-$CH_3$(2) | $CH_2OCH_3$ | $CH_3$ | $-OCO-NH-CH_3$ | Harz |
| 4.15 | α-Naphthyl$(CH_3)_2$(2,3) | $CH_2OCH_3$ | $CH_3$ | $-OCO-NHCH_3$ | Smp. 121–125° |
| 4.16 | α-Naphthyl-$CH_3$(2)Cl(3) | $CH_2OCH_3$ | $CH_3$ | $-OCO-NHCH_3$ | |
| 4.17 | $C_6H_3(CH_3)_2$(2,6) | $CH_2OCH_3$ | $CH_3$ | $-OCO-NHC_5H_{11}$-n | |
| 4.18 | $C_6H_3(C_2H_5)_2$(2,6) | $CH_2OCH_3$ | $CH_3$ | $-OCO-NHC_2H_5$ | |
| 4.19 | $C_6H_3CH_3(2)C_2H_5$(6) | $CH_2OCH_3$ | $CH_3$ | $-OCO-NHCH_3$ | |
| 4.20 | $C_6H_3(CH_3)_2$(2,6) | 2-Tetrahydrofuryl | $CH_3$ | $-OCO-NHCH_3$ | |
| 4.21 | $C_6H_3(CH_3)_2$(2,6) | 2-Tetrahydrofuryl | $CH_3$ | $-OCO-NHC_2H_5$ | |
| 4.22 | $C_6H_3(CH_3)_2$(2,6) | 2-Tetrahydrofuryl | $CH_3$ | $-OCO-NHC_3H_7$-i | |
| 4.23 | $C_6H_3(CH_3)_2$(2,6) | 2-Tetrahydrofuryl | $C_2H_5$ | $-OCO-NHC_2H_5$ | |
| 4.24 | $C_6H_3(CH_3)_2$(2,6) | 2-Tetrahydrofuryl | $C_3H_7$-n | -OCO-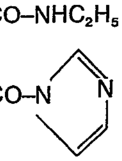 | |

Tabelle 4 (Fortsetzung)

| Verb. Nr. | Ar | $R_1$ | $R_3$ | B | Physik. Konst. |
|---|---|---|---|---|---|
| 4.25 | $C_6H_3(CH_3)_2(2,6)$ | 2-Tetrahydrofuryl | $CH_3$ | 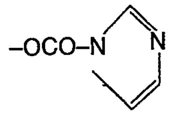 | |
| 4.26 | $C_6H_3(CH_3)_2(2,6)$ | 2-Tetrahydrofuryl | $C_4H_9$–n | 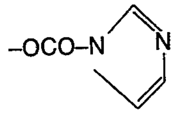 | |
| 4.27 | $C_6H_3(CH_3)_2(2,6)$ | 2-Tetrahydrofuryl | $CH_3$ | $-OCO-NHC_6H_4Cl(4)$ | |
| 4.28 | $C_6H_3(CH_3)_2(2,6)$ | 2-Tetrahydrofuryl | $CH_3$ | $-OCO-NHC_6H_5$ | |
| 4.29 | $C_6H_3(CH_3)_2(2,6)$ | $CH_2OCH_3$ | $CH_3$ | $-OCO-NHC_6H_5$ | |
| 4.30 | $C_6H_3(CH_3)_2(2,6)$ | $CH_2OCH_3$ | $CH_3$ | $-OCO-NHC_6H_4CH_3(4)$ | |
| 4.31 | $C_6H_3(CH_3)_2(2,6)$ | $CH_2OCH_3$ | $CH_3$ | $-OCO-N(CH_3)_2$ | |
| 4.32 | $C_6H_2(CH_3)_3(2,3,6)$ | $CH_2OCH_3$ | $CH_3$ | $-OCO-NHC_6H_5$ | Smp. 138° |
| 4.33 | $C_6H_2(CH_3)_3(2,3,6)$ | $CH_2OCH_3$ | $CH_3$ | $-OCO-NHC_6H_4F(4)$ | Smp. 134° |
| 4.34 | α-Naphthyl$(CH_3)_2$-(2,3)$NO_2$(4) | $CH_2OCH_3$ | $CH_3$ | $-OCO-NHCH_3$ | |
| 4.35 | α-Naphthyl–$(CH_3)_2$-(2,3)$NO_2$(4) | 2-Tetrahydrofuryl | $CH_3$ | $-OCO-NHCH_3$ | |
| 4.36 | $C_6H_3(CH_3)_2(2,6)$ | 2-Furyl | $CH_3$ | $-OCO-NHCH_3$ | |
| 4.37 | $C_6H_3(CH_3)_2(2,6)$ | Cyclopropyl | $CH_3$ | $-OCO-NHC_2H_5$ | |
| 4.38 | $C_6H_3(CH_3)_2(2,6)$ | $-CH=CHCH_3$ | $CH_3$ | $-OCO-NH(CH_3)_2$ | |
| 4.39 | $C_6H_2(CH_3)_2(2,6)Br(4)$ | 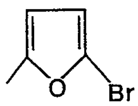 | $C_2H_5$ | $-OCO-NHCH_3$ | |
| 4.40 | α-Naphthyl-$CH_3$(2) | 2-Furyl | $CH_3$ | $-OCO-NHC_3H_7$–n | |
| 4.41 | $C_6H_3(CH_3)_2(2,6)$ | $CH_2OCH_3$ | $CH_3$ | $-OCO-NH(CH_2)_2Cl$ | Harz |
| 4.42 | $C_6H_3(CH_3)_2(2,6)$ | $CH_2OC_2H_5$ | $CH_3$ | $-OCO-NHC_2H_5$ | |
| 4.43 | $C_6H_3(CH_3)_2(2,6)$ | $CH_2OC_2H_5$ | $CH_3$ | $-OCO-N(CH_3)_2$ | |
| 4.44 | $C_6H_3(CH_3)_2(2,6)$ | $CH_2OC_2H_5$ | $CH_3$ | 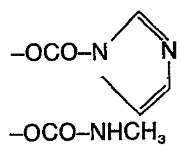 | |
| 4.45 | $C_6H_3Cl(2)OCH_3(6)$ | $CH_2OCH_3$ | $CH_3$ | $-OCO-NHCH_3$ | Harz |
| 4.46 | $C_6H_2(CH_3)_3(2,3,6)$ | 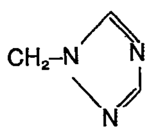 | $CH_3$ | $-OCO-NHC_2H_5$ | |
| 4.47 | $C_6H_3(CH_3)_2(2,6)$ | 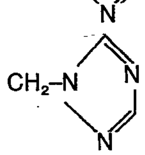 | $CH_3$ | $-OCO-NHCH_3$ | |
| 4.48 | $C_6H_3(CH_3)_2(2,6)$ | 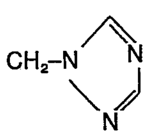 | $CH_3$ | 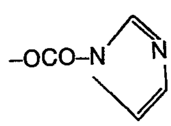 | |
| 4.49 | α-Naphthyl-$CH_3$(2) | 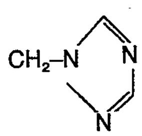 | $CH_3$ | $-OCO-NCH_2H_5$ | |
| 4.50 | α-Naphthyl-$CH_3$(2) | $CH_2OCH_3$ | $CH_3$ | $-SCO-NHC_2H_5$ | |
| 4.51 | $C_6H_3(CH_3)_2(2,6)$ | $CH_2OCH_3$ | $CH_3$ | $-SCS-NHCH_3$ | Harz |
| 4.52 | $C_6H_3(CH_3)_2(2,6)$ | $CH_2OCH_3$ | $CH_3$ | $-SCS-N(CH_3)_2$ | Harz |
| 4.53 | $C_6H_3(CH_3)_2(2,6)$ | $CH_2OCH_3$ | $CH_3$ | $-SCS-N(C_2H_5)_2$ | Harz |
| 4.54 | $C_6H_3(CH_3)_2(2,6)$ | 2-Tetrahydrofuryl | $CH_3$ | $-SCS-NHC_2H_5$ | Viscos |

Tabelle 4 (Fortsetzung)

| Verb. Nr. | Ar | $R_1$ | $R_3$ | B | Physik. Konst. |
|---|---|---|---|---|---|
| 4.55 | $C_6H_3(CH_3)_2(2,6)$ | 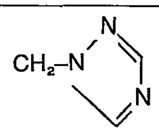 | $CH_3$ | $-SCS-NHCH_3$ | Viscos |
| 4.56 | $C_6H_3(CH_3)_2(2,6)$ | $CH_2OCH_3$ | $CH_3$ | $-OCO-NHC_2H_5$ | Harz |
| 4.57 | α-Naphthyl-$CH_3(2)$ | 2-Tetrahydrofuryl | $CH_3$ | $-OCO-NHCH_3$ | Viscos |
| 4.58 | α-Naphthyl-$CH_3(2)NO_2(4)$ | 2-Tetrahydrofuryl | $CH_3$ | $-OCO-NHCH_3$ | Harz |
| 4.59 | α-Naphthyl-$CH_3(2)NO_2(4)$ | $CH_2OCH_3$ | $CH_3$ | $-OCO-NHCH_3$ | Harz |

Beispiel 5:

a) Herstellung von

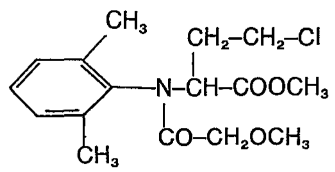

(5.1)

N-(2,6-Dimethylphenyl)-N-methoxyacetyl-2-amino-4-chlor-buttersäuremethylester:

27,7 g
3-[(N-Methoxyacetyl)-N-(2,6-dimethylphenyl)]-amino-tetrahydro-2-furanon
werden in 150 ml Methanol bei 40 bis 50° gelöst, abgekühlt und bei 0 bis 5° mit gasförmigem Chlorwasserstoff gesättigt. Nach 3-tägigem Stehenlassen bei Raumtemperatur wird die Lösung auf 55° erwärmt und 24 Stunden bei dieser Temperatur gehalten. Dann dampft man die Lösung ein, löst den Rückstand in Methylenchlorid, wäscht ihn mit Eiswasser, trocknet die Lösung über Natriumsulfat und dampft sie ein. Nicht umgesetztes Ausgangsmaterial ist in Diethylether unlöslich, wird darin ausgefällt und abfiltriert. Nach Entfernen des Ethers erhält man Kristalle der Verbindung Nr. 5.1, die nach Umkristallisieren aus Benzin bei 70–72° schmelzen.

b) Herstellung von

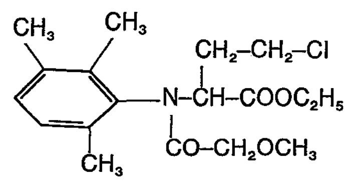

(5.3)

N-(2,3,6-Trimethylphenyl)-N-methoxyacetyl-2-amino-4-chlor-buttersäureethylester:

20,4 g
3-[(N-Methoxyacetyl)-N-(2,3,6-trimethylphenyl)]-aminotetrahydro-2-furanon
werden in 150 ml Ethanol gelöst und unter Rühren tropfenweise mit 12,5 g Thionylchlorid versetzt. Dann wird die Lösung erwärmt und 4 Stunden unter Rückfluss erhitzt, nochmals mit 10 g Thionylchlorid versetzt und weitere 2 Stunden unter Rückfluss erhitzt. Nach dem Eindampfen bleibt ein Harz zurück, das über eine Kieselsäule (Chloroform/Ether 1:1) gereinigt wird. Verbindung Nr. 5.3 fällt als viskoses Harz aus.

Analog zu den Herstellungsvarianten 5a und 5b lassen sich weitere Verbindungen der Formel I herstellen.

Tabelle 5
(B = Hal; $R_2$ = H)

| Verb. Nr. | Ar | B | $R_1$ | $R_3$ | Physik. Konst. |
|---|---|---|---|---|---|
| 5.1 | $C_6H_3(CH_3)_2(2,6)$ | Cl | $CH_2OCH_3$ | $CH_3$ | Smp. 70–72° |
| 5.2 | $C_6H_3(CH_3)_2(2,6)$ | Cl | $CH_2OCH_3$ | $C_2H_5$ | visk. Öl |
| 5.3 | $C_6H_2(CH_3)_3(2,3,6)$ | Cl | $CH_2OCH_3$ | $C_2H_5$ | Harz |
| 5.4 | $C_6H_2(CH_3)_2(2,6)Cl(3)$ | Cl | $CH_2OCH_3$ | $C_2H_5$ | Harz |
| 5.5 | $C_6H_3(CH_3)_2(2,6)$ | Br | $CH_2OCH_3$ | $CH_3$ | |
| 5.6 | $C_6H_3(CH_3)_2(2,6)$ | Br | $CH_2OCH_3$ | $C_2H_5$ | $n_D^{16}$ 1.5326 |
| 5.7 | $C_6H_3(CH_3)_2(2,6)$ | J | $CH_2OCH_3$ | $CH_3$ | |
| 5.8 | $C_6H_3(CH_3)_2(2,6)$ | J | $CH_2OCH_3$ | $C_2H_5$ | |
| 5.9 | $C_6H_3(CH_3)_2(2,6)$ | Cl | $CH_2OCH_3$ | $C_3H_7$–n | |
| 5.10 | $C_6H_3(CH_3)_2(2,6)$ | Cl | $CH_2OCH_3$ | $C_4H_9$–n | |
| 5.11 | $C_6H_3(CH_3)_2(2,6)$ | Cl | $CH_2OC_2H_5$ | $CH_3$ | |
| 5.12 | $C_6H_3(CH_3)_2(2,6)$ | Cl | $CH_2OC_2H_5$ | $C_2H_5$ | |
| 5.13 | $C_6H_3(CH_3)_2(2,6)$ | Cl | 2-Tetrahydrofuryl | $CH_3$ | |
| 5.14 | $C_6H_3(CH_3)_2(2,6)$ | J | 2-Tetrahydrofuryl | $CH_3$ | |
| 5.15 | $C_6H_3(CH_3)_2(2,6)$ | Cl | 2-Tetrahydrofuryl | $C_2H_5$ | |
| 5.16 | $C_6H_3(CH_3)_2(2,6)$ | Cl | 2-Furyl | $CH_3$ | |

Tabelle 5 (Fortsetzung)

| Verb. Nr. | Ar | B | $R_1$ | $R_3$ | Physik. Konst. |
|---|---|---|---|---|---|
| 5.17 | $C_6H_3(CH_3)_2(2,6)$ | Cl | 2-Furyl | $C_2H_5$ | |
| 5.18 | $C_6H_3(CH_3)_2(2,6)$ | Cl | $CH_2SCH_3$ | $CH_3$ | |
| 5.19 | $C_6H_3(CH_3)_2(2,6)$ | Cl | Cyclopropyl | $C_2H_5$ | |
| 5.20 | $C_6H_3(CH_3)_2(2,6)$ | J | Cyclopropyl | $CH_3$ | |
| 5.21 | $C_6H_3(CH_3)_2(2,6)$ | Cl | $-CH=CH-CH_3$ | $CH_3$ | |
| 5.22 | $C_6H_2(CH_3)_3(2,3,6)$ | Br | 2-Tetrahydrofuryl | $CH_3$ | |
| 5.23 | $C_6H_2(CH_3)_2(2,6)Cl(3)$ | Br | 2-Tetrahydrofuryl | $CH_3$ | |
| 5.24 | $C_6H_2(CH_3)_2(2,6)Cl(3)$ | Cl | 2-Tetrahydrofuryl | $C_2H_5$ | |
| 5.25 | $C_6H_2(CH_3)_2(2,6)Cl(3)$ | Cl | 2-Tetrahydrofuryl | $C_4H_9$-n | |
| 5.26 | $C_6H_2(CH_3)_3(2,3,6)$ | Cl | $CH_2OC_2H_5$ | $CH_3$ | |
| 5.27 | $C_6H(CH_3)_4(2,3,5,6)$ | Cl | $CH_2OCH_3$ | $C_2H_5$ | |
| 5.28 | $C_6H_3OCH_3(2)Cl(6)$ | Cl | $CH_2OCH_3$ | $C_2H_5$ | Harz |
| 5.29 | $C_6H_3OCH_3(2)CH_3(6)$ | Cl | $CH_2OCH_3$ | $CH_3$ | |
| 5.30 | $C_6H_3OCH_3(2)CH_3(6)$ | J | $CH_2OCH_3$ | $CH_3$ | |
| 5.31 | $C_6H_2(CH_3)_2(2,6)Br(4)$ | Cl | 2-Furyl | $CH_3$ | |
| 5.32 | α-Naphthyl-$CH_3(2)$ | Cl | 2-Tetrahydrofuryl | $C_2H_5$ | |
| 5.33 | α-Naphthyl-$CH_3(2)$ | Cl | $CH_2OCH_3$ | $C_2H_5$ | Harz |
| 5.34 | α-Naphthyl$(CH_3)_2(2,3)$ | J | $CH_2OC_2H_5$ | $CH_3$ | |
| 5.35 | $C_6H_3(CH_3)_2(2,6)$ | Cl | $CH_2Cl$ | $CH_3$ | |
| 5.36 | $C_6H_3(CH_3)_2(2,6)$ | J | $CH_2J$ | $CH_3$ | |
| 5.37 | $C_6H_3(CH_3)_2(2,6)$ | Br | $CH_3Br$ | $C_2H_5$ | |
| 5.38 | $C_6H(CH_3)_4(2,3,5,6)$ | Cl | $CH_2Cl$ | $C_2H_5$ | |
| 5.39 | α-Naphthyl-$CH_3(2)$ | Cl | $CH_3Cl$ | $C_2H_5$ | |
| 5.40 | α-Naphthyl-$CH_3(2)$ | Cl | $CH_2Cl$ | $CH_3$ | |

Es wurde überraschend gefunden, dass Verbindungen der Formel I ein für praktische Bedürfnisse sehr günstiges Mikrobizid-Spektrum aufweisen. Sie lassen sich beispielsweise zum Schutz von Kulturpflanzen verwenden.

Das Haupteinsatzgebiet von Verbindungen der Formel I liegt in der Bekämpfung von schädlichen Mikroorganismen, vor allem von phytopathogenen Pilzen. So besitzen die Verbindungen der Formel I eine für praktische Bedürfnisse sehr günstige kurative und präventive Wirkung zum Schutz von Kulturpflanzen ohne diese durch unerwünschte Nebenwirkungen zu beeinflussen. Kulturpflanzen seien im Rahmen vorliegender Erfindung beispielsweise: Getreide: (Weizen, Gerste, Roggen, Hafer, Reis); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- und Naturkautschukgewächse sowie Zierpflanzen.

Mit den Wirkstoffen der Formel I können an Pflanzen oder an Pflanzenteilen (Früchte, Blüten, Laubwerk, Stengel, Knollen, Wurzeln) dieser und verwandter Nutzkulturen die auftretenden Mikroorganismen eingedämpft oder vernichtet werden, wobei auch später zuwachsende Pflanzenteile von derartigen Mikroorganismen verschont bleiben. Wirkstoffe der Formel I sind gegen eine ganze Reihe von phytopathogenen Pilzen wirksam, darunter z.B. gegen die zur Familie der Ascomyceten gehörenden Erysiphe- und Venturia-Erreger sowie gegen die zur Klasse der Phycomyceten gehörenden Oomyceten wie Phytophthora, Peronospora, Plasmopara und Pythium. Einige Vertreter der Stoffklassen wirken auch insektizid und bakterizid.

Sie können ferner als Beizmittel zur Behandlung von Saatgut (Früchte, Knollen, Körner) und Pflanzenstecklingen zum Schutz vor Pilzinfektionen sowie gegen im Erdboden auftretende schädliche Mikroorganismen eingesetzt werden.

Die Erfindung betrifft somit ferner die Verwendung der Verbindungen der Formel I zur Bekämpfung phytopathogener Mikroorganismen bzw. zur präventiven Verhütung eines Befalls an Pflanzen.

Zur Bekämpfung dieser Mikroorganismen können die Verbindungen der Formel I für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln. Wirkstoffe der Formel I können auch im Gemisch mit z.B. pestiziden oder pflanzenwuchsverbessernden Präparaten verwendet werden.

Die nachfolgende, beispielhafte Aufzählung soll die Beschaffenheit solcher Mittel näher erläutern.

Der Gehalt an Wirkstoff in handelsfähigen Mitteln liegt zwischen 0,0001 bis 90%.

Zur Applikation können die Verbindungen der Formel I in den folgenden Aufarbeitungsformen vorliegen:

Feste Aufarbeitungsformen:

Stäube- und Streumittel enthalten im allgemeinen bis zu 10% des Wirkstoffs. Ein Stäubemittel kann beispielsweise aus 5 Teilen des Wirkstoffs und 95 Teilen eines Zuschlagstoffes wie Talkum bestehen oder aus 2 Teilen Wirkstoff, 1 Teil hochdisperser Kieselsäure und 97 Teilen Talkum. Darüberhinaus sind weitere Gemische mit solchen und anderen in der Formulierungstechnik gebräuchlichen Trägermaterialien und Zuschlagstoffen denkbar. Bei der Herstellung dieser Stäubemittel werden die Wirkstoffe mit den Träger- und Zuschlagstoffen vermischt und vermahlen und können in dieser Form verstäubt werden.

Granulate wie Umhüllungsgranulate, Imprägniergranulate, Homogengranulate und Pellets [= Körner] enthalten üblicherweise 1 bis 80% des Wirkstoffs. So kann sich ein 5%iges Granulat z.B. aus 5 Teilen des Wirkstoffs, 0,25 Teilen epoxidiertem Pflanzenöl, 0,25 Teilen Cetylpolyglykolether, 3,50 Teilen Polyethylenglykol und 91 Teilen Kaolin (bevorzugte Korngrösse 0,3–0,8 mm) zusammensetzen. Man kann bei der Herstellung des Granulates wie folgt vorgehen:

Die Aktivsubstanz wird mit dem Pflanzenöl vermischt und mit 6 Teilen Aceton gelöst, hierauf wird Polyethylenglykol und Cetylpolyglykolether zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Flüssige Aufarbeitungsformen:

Man unterscheidet im allgemeinen zwischen Wirkstoffkonzentraten, die in Wasser dispergierbar oder löslich sind und Aerosolen. Zu den in Wasser dispergierbaren Wirkstoffkonzentraten zählen z.B. Spritzpulver (wettable powders) und Pasten, die üblicherweise in den Handelspackungen 25–90% und in gebrauchsfertigen Lösungen 0,01 bis 15% des Wirkstoffs enthalten. Emulsionskonzentrate enthalten 10 bis 50% und Lösungskonzentrate enthalten in der gebrauchsfertigen Lösung 0,001 bis 20% Aktivsubstanz. So kann ein 70%iges Spritzpulver z.B. aus 70 Teilen des Wirkstoffs, 5 Teilen Natriumdibutylnaphthylsulfonat, dazu 3 Teilen Naphthalinsulfonsäuren – Phenolsulfonsäuren – Formaldehyd-Kondensat (im Mischungsverhältnis 3:2:1), 10 Teilen Kaolin und 12 Teilen Kreide z.B. Champagne-Kreide zusammengesetzt sein. Ein 40%iges Spritzpulver kann z.B. aus folgenden Stoffen bestehen: 40 Teile Wirkstoff, 5 Teile Natrium-Ligninsulfonat, 1 Teil Natrium-Dibutylnaphthylsulfonat und 54 Teile Kieselsäure. Die Herstellung eines 25%igen Spritzpulvers kann auf unterschiedliche Art erfolgen. So kann dieses sich z.B. zusammensetzen aus: 25 Teilen der Aktivsubstanz, 4,5 Teilen Calcium-Ligninsulfonat, 1,9 Teilen Kreide, z.B. Champagne-Kreide/Hydroxyethylencellulose-Gemisch (1:1), 1,5 Teilen Natrium-Dibutylnaphthylsulfonat, 19,5 Teilen Kieselsäure, 19,5 Teilen Champagne-Kreide und 28,1 Teilen Kaolin. Ein 25%iges Spritzpulver kann z.B. auch bestehen aus 25 Teilen Wirkstoff, 2,5 Teilen Isooctylphenoxy-polyoxyethylen-ethanol, 1,7 Teilen Champagne-Kreide/Hydroxyethylcellulosegemisch (1:1), 8,3 Teilen Natriumsilikat, 16,5 Teilen Kieselgut und 46 Teilen Kaolin. Ein 10%iges Spritzpulver lässt sich z.B. herstellen aus 10 Teilen des Wirkstoffes, 3 Teilen eines Gemisches aus Natriumsalzen von gesättigten Fettalkoholsulfonaten, 5 Teilen Naphthalinsulfonsäure/Formaldehyd-Kondensat und 82 Teilen Kaolin. Andere Spritzpulver können Gemische darstellen aus 5 bis 30% der Aktivsubstanz zusammen mit 5 Teilen eines aufsaugenden Trägermaterials wie Kieselsäure, 55 bis 80 Teilen eines Trägermaterials wie Kaolin und eines Dispergiermittelgemisches, bestehend aus 5 Teilen Natrium-Arylsulfonates sowie aus 5 Teilen eines Alkylarylpolyglykoläthers. Ein 25%iges Emulsions-Konzentrat kann z.B. folgende emulsierbare Stoffe enthalten: 25 Teile des Wirkstoffs, 2,5 Teile epoxidiertes Pflanzenöl, 10 Teile eines Alkylarylsulfonat-Fettalkoholpolyglykolether-Gemisches, 5 Teile Dimethylformamid und 57,5 Teile Xylol.

Aus solchen Konzentraten können durch Verdünnen mit Wasser Emulsionen der gewünschten Anwendungskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen. Solche Mittel gehören ebenfalls zur Erfindung.

Mittel, die wie oben beschrieben, als Wirkkomponente eine Verbindung der Formel I enthalten, beispielsweise Verbindung Nr. 1.6, 1.17, 1.24 bis 1.34, 2.1 bis 2.6, 2.10, 2.19, 2.32 bis 2.48, 3.1, 3.4, 3.9, 3.10, 3.25 bis 3.31, 4.2 bis 4.4, 4.9 oder 4.14 lassen sich mit gutem Erfolg gegen schädliche Mikroorganismen einsetzen.

Biologische Beispiele

Beispiel 6:

Wirkung gegen Erysiphe graminis auf Gerste

Residual-protektive Wirkung

Ca 8 cm hohe Gerstenpflanzen wurden mit einer aus Spritzpulver des Wirkstoffs, wie oben beschrieben hergestellten Spritzbrühe (0,02% Aktivsubstanz wie z.B. eine der Verbindungen aus Ta-

belle 1 bis 5 besprüht. Nach 3–4 Stunden wurden die behandelten Pflanzen mit Konidien des Pilzes bestäubt. Die infizierten Gerstenpflanzen wurden im Gewächshaus bei ca. 22 °C aufgestellt und der Pilzbefall nach 10 Tagen beurteilt. Dabei bewirkten Spritzbrühen, die als Wirkkomponente einen Wirkstoff der Formel I enthielten, z.B. Verbindung Nr. 3.1, 3.2, 4.4, 5.1, 5.3, eine fast vollständige Hemmung des Befalls, im Vergleich zu unbehandelten Kontrollpflanzen.

Beispiel 7:
Wirkung gegen Venturia inaequalis auf Apfel
Residual-protektive Wirkung

Apfelsämlinge mit ca. 5 entwickelten Blättern wurden mit einer aus Spritzpulver, nach einem der obigen Beispiele hergestellten Spritzbrühe (0,06% Aktivsubstanz, wie z.B. eine der Verbindungen aus den Tabellen 1 bis 5) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24 °C aufgestellt. Der Schorfbefall wurde 15 Tage nach der Infektion beurteilt, dabei hemmten Spritzbrühen, die als Aktivsubstanz eine der Verbindungen aus den Tabellen 1 bis 5 (z.B. Verbindung Nr. 2.2, 4.3, 4.9) enthielten, den Pilzbefall fast vollständig.

Beispiel 8:
Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes, wie oben beschrieben hergestellten Spritzbrühe (0,02% Aktivsubstanz, wie z.B. eine der Verbindungen aus den Tabellen 1 bis 5) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90–100% Luftfeuchtigkeit und 20 °C.

b) Residual-kurative Wirkung

Tomatenpflanzen wurden nach 3-wöchiger Anzucht mit einer Sporangiensuspension des Pilzes infiziert. Nach einer Inkubation von 22 Stunden in einer Feuchtkammer bei 90–100% relativer Luftfeuchtigkeit und 20 °C wurden die infizierten Pflanzen getrocknet und mit einer aus Spritzpulver des Wirkstoffes, wie oben beschrieben hergestellten Spritzbrühe (0,02% Aktivsubstanz, wie z.B. eine der Verbindungen aus den Tabellen 1 bis 5) besprüht.

Nach dem Antrocknen des Spritzbelages wurden die behandelten Pflanzen wieder in die Feuchtkammer gebracht. Die Beurteilung des Pilzbefalls erfolgte 5 Tage nach der Infektion.

c) Systemische Wirkung

Zu Tomatenpflanzen wurde nach 3-wöchiger Anzucht eine aus Spritzpulver des Wirkstoffes, wie oben beschrieben hergestellte Spritzbrühe gegossen [0,002% Aktivsubstanz (wie z.B. eine der Verbindungen aus den Tabellen 1 bis 5) bezogen auf das Erdvolumen]. Es wurde darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90–100% relativer Luftfeuchtigkeit und 20 °C.

Die Verbindungen der Formel I zeigten in obigen Versuchen gegenüber Phytophthora-Erregern nicht nur eine hervorragende residual-protektive bzw. residual-kurative sondern zusätzlich eine sehr gute systemische Wirkung. Sie hemmten den Befall auf weniger als 20%. Bei Applikation folgender Verbindungen wurde ein Krankheitsbefall vollständig verhütet: Nr. 1.1, 1.15, 1.17, 1.24 bis 1.34, 2.1 bis 2.5, 2.10, 2.13, 2.19, 2.32 bis 2.48, 3.1, 3.4, 3.9, 3.10, 3.25 bis 3.31, 4.2, 4.4, 4.9, 4.14, 5.1, 5.2.

Beispiel 9:

Wirkung gegen Pythium debaryanum auf Rüben. Wirkung

a) nach Bodenapplikation

Der Pilz wurde auf Karottenschnitzel-Nährlösung kultiviert und einer Erde-Sand-Mischung beigegeben. Die so infizierte Erde wurde in Blumentöpfe abgefüllt und mit Zuckerrübensamen besät. Gleich nach der Aussaat wurden die als Spritzpulver formulierten Versuchspräparate als wässrige Suspensionen über die Erde gegossen (20 ppm einer der Verbindungen aus den Tabellen 1 bis 5 bezogen auf das Erdvolumen). Die Töpfe wurden darauf während 2–3 Wochen im Gewächshaus bei ca. 20 °C aufgestellt. Die Erde wurde dabei durch leichtes Überbrausen stets gleichmässig feucht gehalten. Bei der Auswertung des Tests wurde der Auflauf der Zuckerrübenpflanzen sowie der Anteil gesunder und kranker Pflanzen bestimmt.

b) Wirkung nach Beizapplikation

Der Pilz wurde auf Karottenschnitzel-Nährlösung kultiviert und einer Erde-Sand-Mischung beigegeben. Die so infizierte Erde wurde in Erdschalen abgefüllt und mit Zuckerrübensamen besät, die mit den als Beizpulver formulierten Versuchspräparaten gebeizt worden waren (0,06% einer der Verbindungen aus den Tabellen 1 bis 5).

Die besäten Töpfe wurden während 2–3 Wochen im Gewächshaus bei ca. 20 °C aufgestellt. Die Erde wurde dabei durch leichtes Überbrausen stets gleichmässig feucht gehalten. Bei der Auswertung wurde der Auflauf der Zuckerrübenpflanzen bestimmt. Bei Behandlung mit Verbindungen der Formel I, besonders solchen der Untergruppen Ic und Id, liefen über 85% der Rübenpflanzen auf und hatten ein gesundes Aussehen.

Die Verbindungen Nr. 1.25, 2.1 bis 2.13, 2.19, 2.32 bis 2.48, 3.4, 3.10, 4.2, 4.3, 4.4, 4.9, 4.14, 5.1, 5.2, 5.6 erzielten in obigen Versuchen eine sehr gute Wirkung gegen Pythium-Erreger auf Rüben

(Auflauf der Pflanzen 92–95% wie nicht-infizierte Kontrolle).

Eine gleich gute Wirkung wurde bei analogen Versuchen gegen Pythium-Erregern auf Mais erzielt.

**Patentansprüche für die Vertragsstaaten BE, CH, DE, FR, GB, IT, LI, NL, SE**

1. Homoserin-Derivate der Formel I

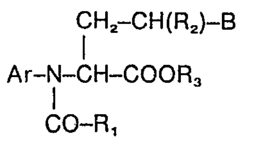

(I),

worin $R_1$ eine gegebenenfalls durch ein Sauerstoffatom unterbrochene aliphatische Kette aus 2 bis 6 Kohlenstoffatomen bedeutet, oder für eine gegebenenfalls durch Halogen substituierte 2-Furyl-, 2-Tetrahydrofuryl, 1H-1,2,4-Triazolylmethyl-, 1-Imidazolylmethyl-, 1-Pyrazolylmethyl, $C_2$–$C_4$-Alkenyl- oder Cyclopropylgruppe steht oder worin, wenn B für Halogen steht, $R_1$ eine Halogenmethylgruppe bedeutet; $R_2$ für Wasserstoff oder Methyl steht, $R_3$ für Wasserstoff oder $C_1$–$C_4$-Alkyl steht und Ar

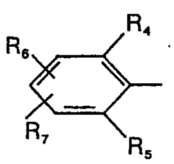 oder 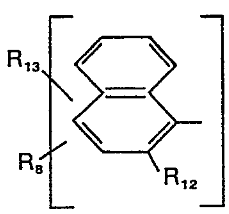 bedeutet,

wobei $R_4$ für $C_1$–$C_3$-Alkyl, $C_1$–$C_3$-Alkoxy oder Halogen, $R_5$ für $C_1$–$C_3$-Alkyl, $C_1$–$C_3$-Alkoxy, $NH_2$, Halogen oder $NO_2$, $R_6$ für Wasserstoff, $NO_2$, $NH_2$, $C_1$–$C_3$-Alkyl, $C_1$–$C_3$-Alkoxy oder Halogen, $R_7$ und $R_8$ für Wasserstoff oder Methyl stehen, $R_{12}$ Methyl, $NO_2$ oder $NH_2$ darstellt, $R_{13}$ für Wasserstoff, Methyl, $NO_2$ oder $NH_2$ steht und B eine der Gruppen –XH, –$XR_9$,

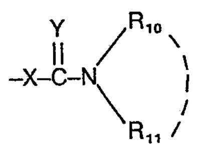

oder Halogen bedeutet, wobei X und Y unabhängig voneinander für Sauerstoff oder Schwefel stehen; $R_9$ für eine gegebenenfalls durch Halogen, $C_1$–$C_3$-Alkoxy oder $C_1$–$C_3$-Alkylthio substituierte $C_1$–$C_5$-Alkylgruppe steht, $R_{10}$ Wasserstoff, Methyl oder Ethyl bedeutet, $R_{11}$ für eine gegebenenfalls durch Halogen substituierte $C_1$–$C_5$-Alkylgruppe oder für einen gegebenenfalls durch Halogen, Methyl, Trifluormethyl oder Nitro substituierten Phenylrest steht oder $R_{10}$ und $R_{11}$ zusammen mit dem Stickstoffatom, an das sie gebunden sind, einen Imidazol- oder 1,2,4-Triazolring bilden.

2. Homoserin-Derivate der Formel I gemäss Anspruch 1, worin Ar

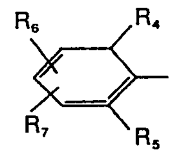 oder 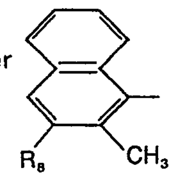 bedeutet,

$R_1$, $R_2$, $R_3$, $R_4$ und B die im Anspruch 1 genannte Bedeutung haben und $R_5$ für $C_1$–$C_3$-Alkyl, $C_1$–$C_3$-Alkoxy oder Halogen, $R_6$ für Wasserstoff, $C_1$–$C_3$-Alkyl, $C_1$–$C_3$-Alkoxy oder Halogen und $R_7$ und $R_8$ für Wasserstoff oder Methyl stehen.

3. Homoserin-Derivate der Formel I gemäss Anspruch 1, worin $R_1$ für $C_1$–$C_3$-Alkoxymethyl, 2-Furyl oder 2-Tetrahydrofuryl steht, $R_2$ und $R_3$ Wasserstoff bedeuten, B für OH oder SH, $R_4$ für $C_1$–$C_3$-Alkyl, $C_1$–$C_3$-Alkoxy oder Halogen, $R_5$ für $C_1$–$C_3$-Alkyl, $C_1$–$C_3$-Alkoxy, Halogen oder Nitro, $R_6$ für Wasserstoff oder $C_1$–$C_3$-Alkyl stehen, $R_7$ Wasserstoff oder Methyl und Ar substituiertes Phenyl bedeuten.

4. Homoserin-Derivate der Formel I, gemäss Anspruch 1, worin $R_1$ für $C_1$–$C_3$-Alkoxymethyl, 2-Furyl oder 2-Tetrahydrofuryl steht, $R_2$ Wasserstoff bedeutet, $R_3$ für $C_1$–$C_3$-Alkyl steht, B für $OR_9$ steht, $R_9$ für eine gegebenenfalls durch Halogen substituierte $C_1$–$C_3$-Alkylgruppe steht, $R_4$ $C_1$–$C_3$-Alkyl, $C_1$–$C_3$-Alkoxy oder Halogen bedeutet, $R_5$ für $C_1$–$C_3$-Alkyl, $C_1$–$C_3$-Alkoxy oder Halogen, $R_6$ für Wasserstoff oder $C_1$–$C_3$-Alkyl stehen, $R_7$ Wasserstoff oder Methyl und Ar substituiertes Phenyl bedeuten.

5. Homoserin-Derivate der Formel I gemäss Anspruch 1, worin Ar eine substituierte Phenylgruppe bedeutet, $R_1$ $C_1$–$C_2$-Alkoxymethyl oder 2-Tetrahydrofuryl darstellt, $R_2$ Wasserstoff ist, $R_3$ für Wasserstoff oder Methyl steht, B wahlweise OH oder Methoxy bedeutet, $R_4$ Methyl ist, $R_5$ für Methyl, Chlor, $NO_2$ oder $NH_2$ steht, $R_6$ Wasserstoff, Methyl, Chlor, $NO_2$ oder $NH_2$ und $R_7$ Wasserstoff oder Methyl bedeuten.

6. Homoserin-Derivate der Formel I gemäss Anspruch 1, worin Ar eine substituierte α-Naphthylgruppe bedeutet, $R_1$ $C_1$–$C_2$-Alkoxymethyl oder 2-Tetrahydrofuryl darstellt, $R_2$ Wasserstoff ist, $R_3$ für Wasserstoff oder Methyl steht, B wahlweise OH oder Methoxy bedeutet, $R_8$ Wasserstoff oder 3-Methyl ist, $R_{12}$ Methyl, $NO_2$ oder $NH_2$ darstellt und $R_{13}$ Wasserstoff, Methyl, $NO_2$ oder $NH_2$ bedeutet.

7. Homoserin-Derivate der Formel I gemäss Anspruch 6, worin $R_{13}$ Wasserstoff, 4-$NO_2$ oder 4-$NH_2$ ist.

8. Homoserin-Derivate der Formel I gemäss Anspruch 5, worin $R_1$ 2-Tetrahydrofuryl bedeutet.

9. Homoserin-Derivate der Formel I gemäss Anspruch 6, worin $R_1$ 2-Tetrahydrofuryl bedeutet.

10. N-(2,6-Dimethylphenyl)-N-methoxyacetyl-homoserin-methylester nach Anspruch 1.

11. N-(2,6-Dimethylphenyl)-N-methoxyacetyl-homoserin-ethylester nach Anspruch 1.

12. N-(2,3,6-Trimethylphenyl)-N-methoxyacetyl-homoserin-methylester nach Anspruch 1.

13. N-(2,6-Dimethylphenyl)-N-methoxyacetyl-[4-(imidazol-1-yl)-carbonyloxy]-buttersäuremethylester nach Anspruch 1.

14. N-(2-Methylnaphthyl)-N-methoxyacetyl--homoserin-methylester nach Anspruch 1.

15. N-(2-Methylnaphthyl)-N-(tetrahydrofurylcarbonyl)-homoserin-methylester nach Anspruch 1.

16. N-(2-Chlor-6-methoxyphenyl)-N-methoxyacetyl-homoserin-methylester nach Anspruch 1.

17. N-(2,6-Dimethyl-3-chlorphenyl)-N-methoxyacetyl-homoserin-methylester nach Anspruch 1.

18. N-(2-Methylnaphthyl)-N-methoxyacetyl-4-(N′-methylcarbamoyloxy)-2-aminobuttersäuremethylester nach Anspruch 1.

19. N-(2,3-Dimethylnaphthyl)-N-methoxyacetylhomoserin-methylester nach Anspruch 1.

20. N-(2-Methyl-6-nitrophenyl)-methoxyacetyl-homoserin-methylester nach Anspruch 1.

21. Mittel zur Bekämpfung und/oder Verhütung eines Befalls durch phytopathogene Mikroorganismen, dadurch gekennzeichnet, dass es als mindestens eine aktive Komponente eine Verbindung der Formel I gemäss Anspruch 1 zusammen mit einem oder mit mehreren Trägerstoffen enthält.

22. Mittel nach Anspruch 21, dadurch gekennzeichnet, dass es als aktive Komponente eine Verbindung der Formel I gemäss Anspruch 2 enthält.

23. Mittel nach Anspruch 21, dadurch gekennzeichnet, dass es als aktive Komponente eine Verbindung der Formel I gemäss Anspruch 3 enthält.

24. Mittel nach Anspruch 21, dadurch gekennzeichnet, dass es als aktive Komponente eine Verbindung der Formel I gemäss Anspruch 4 enthält.

25. Mittel nach Anspruch 21, dadurch gekennzeichnet, dass es als aktive Komponente eine Verbindung der Formel I gemäss einem der Ansprüche 5 bis 20 enthält.

26. Verwendung einer Verbindung der Formel I nach Anspruch 1 zur Bekämpfung und/oder Verhütung eines Befalls durch phytopathogene Mikroorganismen.

27. Verwendung nach Anspruch 26 einer Verbindung der Formel I gemäss Anspruch 2.

28. Verwendung nach Anspruch 26 einer Verbindung der Formel I gemäss einem der Ansprüche 3 bis 20.

29. Verfahren zur Herstellung von Homoserin-Derivaten der Formel I nach Anspruch 1, gekennzeichnet durch alkalische Ringöffnung einer Verbindung der Formel II

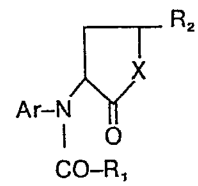

(II)

in einem polaren Lösungsmittel mit Hilfe eines Alkali- oder Erdalkalihydroxids bei −10° bis +100 °C, wobei Ar, $R_1$, $R_2$ und X die für Formel I gegebene Bedeutung haben, und, falls gewünscht, weitere Einführung eines für $R_3$ oder B genannten Substituenten.

**Patentansprüche für den Vertragsstaat AT**

1. Mittel zur Bekämpfung und/oder Verhütung eines Befalls durch phytopathogene Mikroorganismen, dadurch gekennzeichnet, dass es neben einem oder mehreren Trägerstoffen als mindestens eine aktive Komponente ein Homoserin-Derivat der Formel I enthält

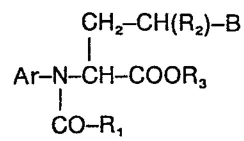

(I),

worin $R_1$ eine gegebenenfalls durch ein Sauerstoffatom unterbrochene aliphatische Kette aus 2 bis 6 Kohlenstoffatomen bedeutet, oder für eine gegebenenfalls durch Halogen substituierte 2-Furyl-, 2-Tetrahydrofuryl-, 1H-1,2,4-Triazolylmethyl-, 1-Imidazolylmethyl-, 1-Pyrazolylmethyl-, $C_2$–$C_4$-Alkenyl- oder Cyclopropylgruppe steht oder worin, wenn B für Halogen steht, $R_1$ eine Halogenmethylgruppe bedeutet; $R_2$ für Wasserstoff oder Methyl steht, $R_3$ für Wasserstoff oder $C_1$–$C_4$-Alkyl steht und Ar

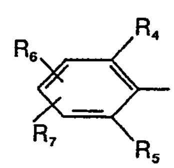 oder 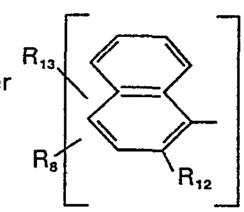 bedeutet,

wobei $R_4$ für $C_1$–$C_3$-Alkyl, $C_1$–$C_3$-Alkoxy oder Halogen, $R_5$ für $C_1$–$C_3$-Alkyl, $C_1$–$C_3$-Alkoxy, $NH_2$, Halogen oder $NO_2$, $R_6$ für Wasserstoff, $NO_2$, $NH_2$, $C_1$–$C_3$-Alkyl, $C_1$–$C_3$-Alkoxy oder Halogen, $R_7$ und $R_8$ für Wasserstoff oder Methyl stehen, $R_{12}$ Methyl, $NO_2$ oder $NH_2$ darstellt, $R_{13}$ für Wasserstoff, Methyl, $NO_2$ oder $NH_2$ steht und B eine der Gruppen $-XH$, $-XR_9$,

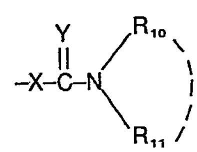 oder

Halogen bedeutet, wobei X und Y unabhängig voneinander für Sauerstoff oder Schwefel stehen; $R_9$ für eine gegebenenfalls durch Halogen, $C_1$–$C_3$-Alkoxy oder $C_1$–$C_3$-Alkylthio substituierte $C_1$–$C_5$-

Alkylgruppe steht, $R_{10}$ Wasserstoff, Methyl oder Ethyl bedeutet, $R_{11}$ für eine gegebenenfalls durch Halogen substituierte $C_1$–$C_5$-Alkylgruppe oder für einen gegebenenfalls durch Halogen, Methyl, Trifluormethyl oder Nitro substituierten Phenylrest steht oder $R_{10}$ und $R_{11}$ zusammen mit dem Stickstoffatom, an das sie gebunden sind, einen Imidazol- oder 1,2,4-Triazolring bilden.

2. Mittel gemäss Anspruch 1, worin in der Formel I

Ar 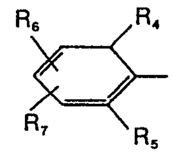 oder 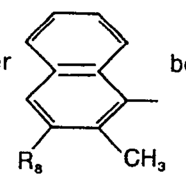 bedeutet,

$R_1$, $R_2$, $R_3$, $R_4$ und B die im Anspruch 1 genannte Bedeutung haben und $R_5$ für $C_1$–$C_3$-Alkyl, $C_1$–$C_3$-Alkoxy oder Halogen, $R_6$ für Wasserstoff, $C_1$–$C_3$-Alkyl, $C_1$–$C_3$-Alkoxy oder Halogen und $R_7$ und $R_8$ für Wasserstoff oder Methyl stehen.

3. Mittel gemäss Anspruch 1, worin in der Formel I $R_1$ für $C_1$–$C_3$-Alkoxymethyl, 2-Furyl oder 2-Tetrahydrofuryl steht, $R_2$ und $R_3$ Wasserstoff bedeuten, B für OH oder SH, $R_4$ für $C_1$–$C_3$-Alkyl, $C_1$–$C_3$-Alkoxy oder Halogen, $R_5$ für $C_1$–$C_3$-Alkyl, $C_1$–$C_3$-Alkoxy, Halogen oder Nitro, $R_6$ für Wasserstoff oder $C_1$–$C_3$-Alkyl stehen, $R_7$ Wasserstoff oder Methyl und Ar substituiertes Phenyl bedeuten.

4. Mittel gemäss Anspruch 1, worin in der Formel I $R_1$ für $C_1$–$C_3$-Alkoxymethyl, 2-Furyl oder 2-Tetrahydrofuryl steht, $R_2$ Wasserstoff bedeutet, $R_3$ für $C_1$–$C_3$-Alkyl steht, B für $OR_9$ steht, $R_9$ für eine gegebenenfalls durch Halogen substituierte $C_1$–$C_3$-Alkylgruppe steht, $R_4$ $C_1$–$C_3$-Alkyl, $C_1$–$C_3$-Alkoxy oder Halogen bedeutet, $R_5$ für $C_1$–$C_3$-Alkyl, $C_1$–$C_3$-Alkoxy oder Halogen, $R_6$ für Wasserstoff oder $C_1$–$C_3$-Alkyl stehen, $R_7$ Wasserstoff oder Methyl und Ar substituiertes Phenyl bedeuten.

5. Mittel gemäss Anspruch 1, worin in der Formel I Ar eine substituierte Phenylgruppe bedeutet, $R_1$ $C_1$–$C_2$-Alkoxymethyl oder 2-Tetrahydrofuryl darstellt, $R_2$ Wasserstoff ist, $R_3$ für Wasserstoff oder Methyl steht, B wahlweise OH oder Methoxy bedeutet, $R_4$ Methyl ist, $R_5$ für Methyl, Chlor, $NO_2$ oder $NH_2$ steht, $R_6$ Wasserstoff, Methyl, Chlor, $NO_2$ oder $NH_2$ und $R_7$ Wasserstoff oder Methyl bedeuten.

6. Mittel gemäss Anspruch 1, worin in der Formel I Ar eine substituierte α-Naphthylgruppe bedeutet, $R_1$ $C_1$–$C_2$-Alkoxymethyl oder 2-Tetrahydrofuryl darstellt, $R_2$ Wasserstoff ist, $R_3$ für Wasserstoff oder Methyl steht, B wahlweise OH oder Methoxy bedeutet, $R_8$ Wasserstoff oder 3-Methyl ist, $R_{12}$ Methyl, $NO_2$ oder $NH_2$ darstellt und $R_{13}$ Wasserstoff, Methyl, $NO_2$ oder $NH_2$ bedeutet.

7. Mittel gemäss Anspruch 6, worin in der Formel I $R_{13}$ Wasserstoff, 4-$NO_2$ oder 4-$NH_2$ ist.

8. Mittel gemäss Anspruch 5, worin in der Formel I $R_1$ 2-Tetrahydrofuryl bedeutet.

9. Mittel gemäss Anspruch 6, worin in der Formel I $R_1$ 2-Tetrahydrofuryl bedeutet.

10. Mittel gemäss Anspruch 1, enthaltend N-(2,6-Dimethylphenyl)-N-methoxyacetyl-homoserinmethylester.

11. Mittel gemäss Anspruch 1, enthaltend N-(2,6-Dimethylphenyl)-N-methoxyacetyl-homoserinethylester.

12. Mittel gemäss Anspruch 1, enthaltend N-(2,3,6-Trimethylphenyl)-N-methoxyacetyl-homoserin-methylester.

13. Mittel gemäss Anspruch 1, enthaltend N-(2,6-Dimethylphenyl)-N-methoxyacetyl-[4-(imidazol-1-yl)-carbonyloxy]-buttersäuremethylester.

14. Mittel gemäss Anspruch 1, enthaltend N-(2-Methylnaphthyl)-N-methoxyacetyl-homoserinmethylester.

15. Mittel gemäss Anspruch 1, enthaltend N-(2-Methylnaphthyl)-N-(tetrahydrofurylcarbonyl)-homoserin-methylester.

16. Mittel gemäss Anspruch 1, enthaltend N-2-Chlor-6-methoxyphenyl)-N-methoxyacetyl--homoserin-methylester.

17. Mittel gemäss Anspruch 1, enthaltend N-(2,6-Dimethyl-3-chlorphenyl)-N-methoxyacetyl-homoserin-methylester.

18. Mittel gemäss Anspruch 1, enthaltend N-(2-Methylnaphthyl)-N-methoxyacetyl-4-(N′-methylcarbamoyloxy)-2-aminobuttersäure-methylester.

19. Mittel gemäss Anspruch 1, enthaltend N-(2,3-Dimethylnaphthyl)-N-methoxyacetyl-homoserin-methylester.

20. Mittel gemäss Anspruch 1, enthaltend N-(2-Methyl-6-nitrophenyl)-N-methoxyacetyl-homo-serin-methylester.

21. Verwendung einer Verbindung der Formel I nach Anspruch 1 zur Bekämpfung und/oder Verhütung eines Befalls durch phytopathogene Mikroorganismen.

22. Verwendung nach Anspruch 21 einer Verbindung der Formel I gemäss Anspruch 2.

23. Verwendung nach Anspruch 21 einer Verbindung der Formel I gemäss einem der Ansprüche 3 bis 20.

24. Verfahren zur Herstellung von Homoserin-Derivaten der Formel I nach Anspruch 1, gekennzeichnet durch alkalische Ringöffnung einer Verbindung der Formel II

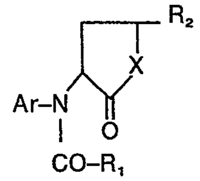 (II)

in einem polaren Lösungsmittel mit Hilfe eines Alkali- oder Erdalkalihydroxids bei −10° bis +100 °C, wobei Ar, $R_1$, $R_2$ und X die für Formel I gegebene Bedeutung haben, und, falls gewünscht, weitere Einführung eines für $R_3$ oder B genannten Substituenten.

**Claims for the Contracting States: BE, CH, DE, FR, GB, IT, LI, NL, SE**

1. A homoserine derivative of the formula I

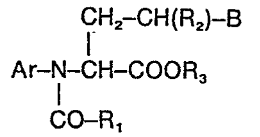

(I),

wherein $R_1$ is an aliphatic chain of 2 to 6 carbon atoms which may be interrupted by an oxygen atom, or $R_1$ is a 2-furyl, 2-tetrahydrofuryl, 1H-1,2,4-triazolylmethyl, 1-imidazolylmethyl, 1-pyrazolylmethyl, $C_2$–$C_4$-alkenyl or cyclopropyl group, each of which is unsubstituted or is substituted by halogen, or wherein, when B is halogen, $R_1$ is a halomethyl group; $R_2$ is hydrogen or methyl, $R_3$ is hydrogen or $C_1$–$C_4$-alkyl,

and Ar is 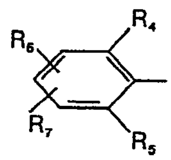 or 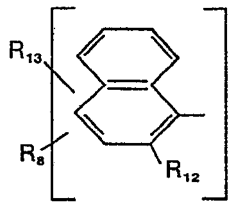 ,

in which $R_4$ is $C_1$–$C_3$-alkyl, $C_1$–$C_3$-alkoxy or halogen, $R_5$ is $C_1$–$C_3$-alkyl, $C_1$–$C_3$-alkoxy, $NH_2$, halogen or $NO_2$, $R_6$ is hydrogen, $NO_2$, $NH_2$, $C_1$–$C_3$-alkyl, $C_1$–$C_3$-alkoxy or halogen, $R_7$ and $R_8$ are hydrogen or methyl, $R_{12}$ is methyl, $NO_2$ or $NH_2$, $R_{13}$ is hydrogen, methyl, $NO_2$ or $NH_2$, and B is one of the following groups

–XH, –$XR_9$ 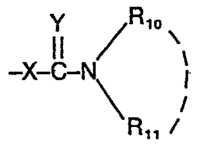

or halogen,

wherein each of X and Y independently of the other is oxygen or sulfur; $R_9$ is a $C_1$–$C_5$-alkyl group which is unsubstituted or substituted by halogen, $C_1$–$C_3$-alkoxy or $C_1$–$C_3$-alkylthio, $R_{10}$ is hydrogen, methyl or ethyl, $R_{11}$ is a $C_1$–$C_5$-alkyl group which is unsubstituted or substituted by halogen, or $R_{11}$ is a phenyl group which is unsubstituted or substituted by halogen, methyl, trifluoromethyl or nitro, or $R_{10}$ and $R_{11}$ together with the nitrogen atom to which they are bound form an imidazole or 1,2,4-triazole ring.

2. A homoserine derivative of the formula I according to claim 1, wherein

Ar is 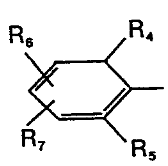 or 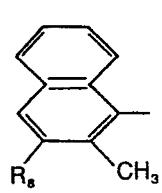 ,

$R_1$, $R_2$, $R_3$, $R_4$ and B are as defined in claim 1, and $R_5$ is $C_1$–$C_3$-alkyl, $C_1$–$C_3$-alkoxy or halogen, $R_6$ is hydrogen, $C_1$–$C_3$-alkyl, $C_1$–$C_3$-alkoxy or halogen, and $R_7$ and $R_8$ are hydrogen or methyl.

3. A homoserine derivative of the formula I according to claim 1, wherein $R_1$ is $C_1$–$C_3$-alkoxymethyl, 2-furyl or 2-tetrahydrofuryl, $R_2$ and $R_3$ are hydrogen, B is OH or SH, $R_4$ is $C_1$–$C_3$-alkyl, $C_1$–$C_3$-alkoxy or halogen, $R_5$ is $C_1$–$C_3$-alkyl, $C_1$–$C_3$-alkoxy, halogen or nitro, $R_6$ is hydrogen or $C_1$–$C_3$-alkyl, $R_7$ is hydrogen or methyl, and Ar is substituted phenyl.

4. A homoserine derivative of the formula I according to claim 1, wherein $R_1$ is $C_1$–$C_3$-alkoxymethyl, 2-furyl or 2-tetrahydrofuryl, $R_2$ is hydrogen, $R_3$ is $C_1$–$C_3$-alkyl, B is $OR_9$, $R_9$ is a $C_1$–$C_3$-alkyl group which is unsubstituted or substituted by halogen, $R_4$ is $C_1$–$C_3$-alkyl, $C_1$–$C_3$-alkoxy or halogen, $R_5$ is $C_1$–$C_3$-alkyl, $C_1$–$C_3$-alkoxy or halogen, $R_6$ is hydrogen or $C_1$–$C_3$-alkyl, $R_7$ is hydrogen or methyl, and Ar is substituted phenyl.

5. A homoserine derivative of the formula I according to claim 1, wherein Ar is a substituted phenyl group, $R_1$ is $C_1$–$C_2$-alkoxymethyl or 2-tetrahydrofuryl, $R_2$ is hydrogen, $R_3$ is hydrogen or methyl, B is either OH or methoxy, $R_4$ is methyl, $R_5$ is methyl, chlorine, $NO_2$ or $NH_2$, $R_6$ is hydrogen, methyl, chlorine, $NO_2$ or $NH_2$, and $R_7$ is hydrogen or methyl.

6. A homoserine derivative of the formula I according to claim 1, wherein Ar is a substituted α-naphthyl group, $R_1$ is $C_1$–$C_2$-alkoxymethyl or 2-tetrahydrofuryl, $R_2$ is hydrogen, $R_3$ is hydrogen or methyl, B is either OH or methoxy, $R_8$ is hydrogen or 3-methyl, $R_{12}$ is methyl, $NO_2$ or $NH_2$, and $R_{13}$ is hydrogen, methyl, $NO_2$ or $NH_2$.

7. A homoserine derivative of the formula I according to claim 6, wherein $R_{13}$ is hydrogen, 4-$NO_2$ or 4-$NH_2$.

8. A homoserine derivative of the formula I according to claim 5, wherein $R_1$ is 2-tetrahydrofuryl.

9. A homoserine derivative of the formula I according to claim 6, wherein $R_1$ is 2-tetrahydrofuryl.

10. N-(2,6-Dimethylphenyl)-N-methoxyacetyl-homoserine-methyl ester
according to claim 1.

11. N-(2,6-Dimethylphenyl)-N-methoxyacetyl-homoserine-ethyl ester
according to claim 1.

12. N-2,3,6-Trimethylphenyl)-N-methoxyacetyl-homoserine-methyl ester
according to claim 1.

13. N-(2,6-Dimethylphenyl)-N-methoxyacetyl-[4-(imidazol-1-yl)carbonyloxy]butyric acid methyl ester
according to claim 1.

14. N-(2-Methylnaphthyl)-N-methoxyacetyl-homoserine-methyl ester
according to claim 1.

15. N-(2-Methylnaphthyl)-N-(tetrahydrofuryl-carbonyl)-homoserine-methyl ester
according to claim 1.

16. N-(2-Chloro-6-methoxyphenyl)-N-methoxyacetyl-homoserine-methyl ester
according to claim 1.

17. N-(2,6-Dimethyl-3-chlorophenyl)-N-methoxyacetyl-homoserine-methyl ester
according to claim 1.

18. N-(2-Methylnaphthyl)-N-methoxyacetyl-4-(N'-methylcarbamoyloxy)-2-aminobutyric acid methyl ester
according to claim 1.

19. N-(2,3-Dimethylnaphthyl)-N-methoxyacetyl-homoserine-methyl ester
according to claim 1.

20. N-(2-Methyl-6-nitrophenyl)-N-methoxyacetyl-homoserine-methyl ester
according to claim 1.

21. A composition for controlling phytopathogenic microorganisms and/or for preventing infection by said phytopathogenic microorganisms, which composition contains as at least one active ingredient a compound of the formula I according to claim 1, together with one or more carrier substances.

22. A composition according to claim 21, which contains as active ingredient a compound of the formula I according to claim 2.

23. A composition according to claim 21, which contains as active ingredient a compound of the formula I according to claim 3.

24. A composition according to claim 21, which contains as active ingredient a compound of the formula I according to claim 4.

25. A composition according to claim 21, which contains as active ingredient a compound of the formula I according to any one of claims 5 to 20.

26. A method of controlling phytopathogenic microorganisms and/or of preventing infection by said phytopathogenic microorganisms, which method comprises applying to the locus to be protected an effective amount of a compound of the formula I according to claim 1.

27. A method according to claim 26 which comprises applying an effective amount of a compound of the formula I according to claim 2.

28. A method according to claim 26 which comprises applying an effective amount of a compound of the formula I according to any one of claims 3 to 20.

29. A process for the preparation of a homoserine derivative of the formula I according to claim 1, which process comprises effecting alkaline ring opening of a compound of the formula II

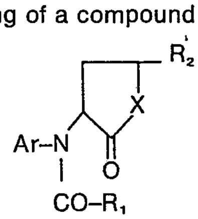

(II)

in a polar solvent by means of an alkali metal hydroxide or alkaline earth metal hydroxide at a temperature in the range from $-10°$ to $+100\,°C$, in which formula II Ar, $R_1$, $R_2$ and X are as defined for the formula I, and, if desired, further introducing a substituent indicated for $R_3$ or B.

**Claims for the Contracting State: AT**

1. A composition for controlling phytopathogenic microorganisms and/or for preventing infection by said phytopathogenic microorganisms, which composition contains as at least one active ingredient a homoserine derivative of the formula I

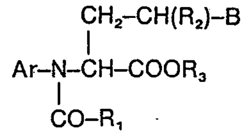

(I),

wherein $R_1$ is an aliphatic chain of 2 to 6 carbon atoms which may be interrupted by an oxygen atom, or $R_1$ is a 2-furyl, 2-tetrahydrofuryl, 1H-1,2,4-triazolylmethyl, 1-imidazolylmethyl, 1-pyrazolylmethyl, $C_2$–$C_4$-alkenyl or cyclopropyl group, each of which is unsubstituted or is substituted by halogen, or wherein, when B is halogen, $R_1$ is a halomethyl group; $R_2$ is hydrogen or methyl, $R_3$ is hydrogen or $C_1$–$C_4$-alkyl, and Ar is

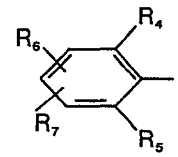

or

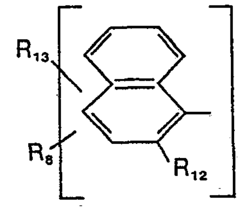

,

in which $R_4$ is $C_1$–$C_3$-alkyl, $C_1$–$C_3$-alkoxy or halogen, $R_5$ is $C_1$–$C_3$-alkyl, $C_1$–$C_3$-alkoxy, $NH_2$, halogen or $NO_2$, $R_6$ is hydrogen, $NO_2$, $NH_2$, $C_1$–$C_3$-alkyl, $C_1$–$C_3$-alkoxy or halogen, $R_7$ and $R_8$ are hydrogen or methyl, $R_{12}$ is methyl, $NO_2$ or $NH_2$, $R_{13}$ is hydrogen, methyl, $NO_2$ or $NH_2$, and B is one of the following groups

$-XH$, $-XR_9$ 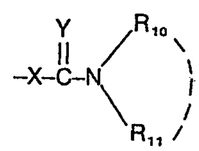

or halogen,

wherein each of X and Y independently of the other is oxygen or sulfur; $R_9$ is a $C_1$–$C_5$-alkyl group which is unsubstituted or substituted by halogen, $C_1$–$C_3$-alkoxy or $C_1$–$C_3$-alkylthio, $R_{10}$ is hydrogen, methyl or ethyl, $R_{11}$ is a $C_1$–$C_5$-alkyl group which is unsubstituted or substituted by halogen, or $R_{11}$ is a phenyl group which is unsubstituted or substituted by halogen, methyl, trifluoromethyl or nitro, or $R_{10}$ and $R_{11}$ together with the nitrogen atom to which they are bound form an imidazole or 1,2,4-triazole ring, together with one or more carrier substances.

2. A composition according to claim 1, wherein in the formula I

Ar is 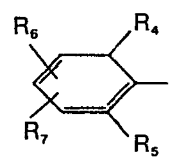 or 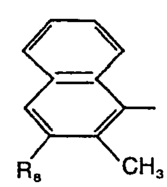 ,

$R_1$, $R_2$, $R_2$, $R_4$ and B are as defined in claim 1, and $R_5$ is $C_1$–$C_3$-alkyl, $C_1$–$C_3$-alkoxy or halogen, $R_6$ is hydrogen, $C_1$–$C_3$-alkyl, $C_1$–$C_3$-alkoxy or halogen, and $R_7$ and $R_8$ are hydrogen or methyl.

3. A composition according to claim 1, wherein in the formula I $R_1$ is $C_1$–$C_3$-alkoxymethyl, 2-furyl or 2-tetrahydrofuryl, $R_2$ and $R_3$ are hydrogen, B is OH or SH, $R_4$ is $C_1$–$C_3$-alkyl, $C_1$–$C_3$-alkoxy or halogen, $R_5$ is $C_1$–$C_3$-alkyl, $C_1$–$C_3$-alkoxy, halogen or nitro, $R_6$ is hydrogen or $C_1$–$C_3$-alkyl, $R_7$ is hydrogen or methyl, and Ar is substituted phenyl.

4. A composition according to claim 1, wherein in the formula I $R_1$ is $C_1$–$C_3$-alkoxymethyl, 2-furyl or 2-tetrahydrofuryl, $R_2$ is hydrogen, $R_3$ is $C_1$–$C_3$-alkyl, B is $OR_9$, $R_9$ is a $C_1$–$C_3$-alkyl group which is unsubstituted or substituted by halogen, $R_4$ is $C_1$–$C_3$-alkyl, $C_1$–$C_3$-alkoxy or halogen, $R_5$ is $C_1$–$C_3$-alkyl, $C_1$–$C_3$-alkoxy or halogen, $R_6$ is hydrogen or $C_1$–$C_3$-alkyl, $R_7$ is hydrogen or methyl, and Ar is substituted phenyl.

5. A composition according to claim 1, wherein in the formula I Ar is a substituted phenyl group, $R_1$ is $C_1$–$C_2$-alkoxymethyl or 2-tetrahydrofuryl, $R_2$ is hydrogen, $R_3$ is hydrogen or methyl, B is either OH or methoxy, $R_4$ is methyl, $R_5$ is methyl, chlorine, $NO_2$ or $NH_2$, $R_6$ is hydrogen, methyl, chlorine, $NO_2$ or $NH_2$, and $R_7$ is hydrogen or methyl.

6. A composition according to claim 1, wherein in the formula I Ar is a substituted α-naphthyl group, $R_1$ is $C_1$–$C_2$-alkoxymethyl or 2-tetrahydrofuryl, $R_2$ is hydrogen, $R_3$ is hydrogen or methyl, B is either OH or methoxy, $R_8$ is hydrogen or 3-methyl, $R_{12}$ is methyl, $NO_2$ or $NH_2$, and $R_{13}$ is hydrogen, methyl, $NO_2$ or $NH_2$.

7. A composition according to claim 6, wherein in the formula I $R_{13}$ is hydrogen, 4-$NO_2$ or 4-$NH_2$.

8. A composition according to claim 5, wherein in the formula I $R_1$ is 2-tetrahydrofuryl.

9. A composition according to claim 6, wherein in the formula I $R_1$ is 2-tetrahydrofuryl.

10. A composition according to claim 1, containing
N-(2,6-dimethylphenyl)-N-methoxyacetyl-homoserine-methyl ester.

11. A composition according to claim 1, containing
N-(2,6-dimethylphenyl)-N-methoxyacetyl-homoserine-ethyl ester.

12. A composition according to claim 1, containing
N-(2,3,6-trimethylphenyl)-N-methoxyacetyl-homoserine-methyl ester.

13. A composition according to claim 1, containing
N-(2,6-dimethylphenyl)-N-methoxyacetyl-[4-(imidazol-1-yl)-carbonyloxy]butyric acid methyl ester.

14. A composition according to claim 1, containing
N-(2-methylnaphthyl)-N-methoxyacetyl-homoserine-methyl ester.

15. A composition according to claim 1, containing
N-(2-methylnaphthyl)-N-(tetrahydrofurylcarbonyl)-homoserine-methyl ester.

16. A composition according to claim 1, containing
N-(2-chloro-6-methoxyphenyl)-N-methoxyacetyl-homoserine-methyl ester.

17. A composition according to claim 1, containing
N-(2,6-dimethyl-3-chlorophenyl)-N-methoxyacetylhomoserine-methyl ester.

18. A composition according to claim 1, containing
N-(2-methylnaphthyl)-N-methoxyacetyl-4-(N'-methylcarbamoyloxy)-2-aminobutyric acid methyl ester.

19. A composition according to claim 1, containing
N-(2,3-dimethylnaphthyl)-N-methoxyacetyl-homoserine-methyl ester.

20. A composition according to claim 1, containing
N-(2-methyl-6-nitrophenyl)-N-methoxyacetyl-homoserine-methyl ester.

21. A method of controlling phytopathogenic microorganisms and/or of preventing infection by said phytopathogenic microorganisms, which method comprises applying to the locus to be protected an effective amount of a compound of the formula I according to claim 1.

22. A method according to claim 21 which comprises applying an effective amount of a compound of the formula I according to claim 2.

23. A method according to claim 21 which comprises applying an effective amount of a compound of the formula I according to any one of claims 3 to 20.

24. A process for the preparation of a homoserine derivative of the formula I according to claim 1, which process comprises effecting alkaline ring opening of a compound of the formula II

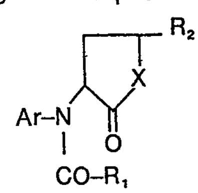

(II)

in a polar solvent by means of an alkali metal hydroxide or alkaline earth metal hydroxide at a temperature in the range from −10° to +100 °C, in which formula II Ar, $R_1$, $R_2$ and X are as defined for the formula I, and, if desired, further introducing a substituent indicated for $R_3$ or B.

**Revendications pour les Etats contractants: BE, CH, DE, FR, GB, IT, LI, NL, SE**

1. Détivés de l'homosérine de formule I

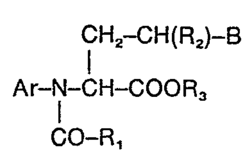

(I),

dans laquelle $R_1$ représente une chaîne aliphatique en C 2–C 6 éventuellement interrompue par un atome d'oxygène, ou un groupe 2-furyle, 2-tétrahydrofuryle, 1H-1,2,4-triazolylméthyle, 1-imidazolylméthyle, 1-pyrazolylméthyle, alcényle en C 2–C 4 ou cyclopropyle éventuellement substitué par des halogènes ou dans laquelle, lorsque B représente un halogène, $R_1$ représente un groupe halogénométhyle; $R_2$ représente l'hydrogène ou un groupe méthyle, $R_3$ représente l'hydrogène ou un groupe alkyle en C 1–C 4 et Ar représente un groupe

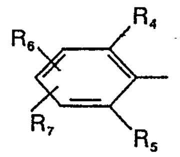

ou

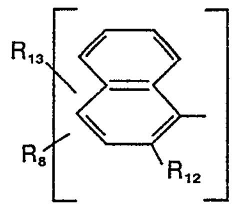

dans lequel $R_4$ représente un groupe alkyle en C 1–C 3, alcoxy en C 1–C 3 ou un halogène, $R_5$ représente un groupe alkyle en C 1–C 3, alcoxy en C 1–C 3, $NH_2$, un halogène ou un groupe $NO_2$, $R_6$ représente l'hydrogène, un groupe $NO_2$, $NH_2$, alkyle en C 1–C 3, alcoxy en C 1–C 3 ou un halogène, $R_7$ et $R_8$ représentent l'hydrogène ou des groupes méthyle, $R_{12}$ représente un groupe méthyle, $NO_2$ ou $NH_2$, $R_{13}$ représente l'hydrogène, un groupe méthyle, $NO_2$ ou $NH_2$ et B représente l'un des groupes –XH, $-XR_9$,

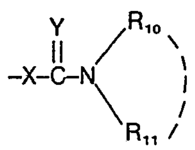

ou un halogène, X et Y représentant chacun, indépendamment l'un de l'autre, l'oxygène ou le soufre; $R_9$ représente un groupe alkyle en C 1–C 5 éventuellement substitué par des halogènes, des groupes alcoxy en C 1–C 3 ou alkylthio en C 1–C 3, $R_{10}$ représente l'hydrogène, un groupe méthyle ou éthyle, $R_{11}$ représente un groupe alkyle en C 1–C 5 éventuellement substitué par des halogènes ou un groupe phényle éventuellement substitué par des halogènes, des groupes méthyle, trifluorométhyle ou nitro, ou bien $R_{10}$ et $R_{11}$ forment ensemble et avec l'atome d'azote auquel ils sont reliés un cycle imidazole ou 1,2,4-triazole.

2. Dérives de l'homosérine de formule I selon la revendication 1, dans laquelle Ar représente

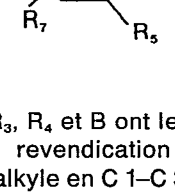

ou

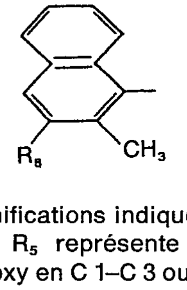

$R_1$, $R_2$, $R_3$, $R_4$ et B ont les significations indiquées dans la revendication 1 et $R_5$ représente un groupe alkyle en C 1–C 3, alcoxy en C 1–C 3 ou un halogène, $R_6$ représente l'hydrogène, un groupe alkyle en C 1–C 3, alcoxy en C 1–C 3 ou un halogène et $R_7$ et $R_8$ représentent l'hydrogène ou des groupes méthyle.

3. Dérivés de l'homosérine de formule I selon la revendication 1, dans laquelle $R_1$ représente un groupe (alcoxy en C 1–C 3)-méthyle, 2-furyle ou 2-tétrahydrofuryle, $R_2$ et $R_3$ représentent l'hydrogène, B représente OH ou SH, $R_4$ représente un groupe alkyle en C 1–C 3, alcoxy en C 1–C 3 ou un halogène, $R_5$ représente un groupe alkyle en C 1–C 3, alcoxy en C 1–C 3, un halogène ou un groupe nitro, $R_6$ représente l'hydrogène ou un groupe alkyle en C 1–C 3, $R_7$ représente l'hydrogène ou un groupe méthyle et Ar représente un groupe phényle substitué.

4. Dérivés de l'homosérine de formule I selon la revendication 1, dans laquelle $R_1$ représente un groupe (alcoxy en C 1–C 3)-méthyle, 2-furyle ou 2-tétrahydrofuryle, $R_2$ représente l'hydrogène, $R_3$ représente un groupe alkyle en C 1–C 3, B représente $OR_9$, $R_9$ représente un groupe alkyle en C 1–C 3 éventuellement substitué par des halogènes, $R_4$ représente un groupe alkyle en C 1–C 3, alcoxy en C 1–C 3 ou un halogène, $R_5$ représente un groupe alkyle en C 1–C 3, alcoxy en C 1–C 3 ou un halogène, $R_6$ représente l'hydrogène ou un groupe alkyle en C 1–C 3, $R_7$ représente l'hydrogène au un groupe méthyle et Ar représente un groupe phényle substitué.

5. Dérivés de l'homosérine de formule I selon la revendication 1, dans laquelle Ar représente un groupe phényle substitué, $R_1$ représente un groupe (alcoxy en C 1–C 2)-méthyle ou 2-tétrahydrofuryle, $R_2$ représente l'hydrogène, $R_3$ représente l'hydrogène ou un groupe méthyle, B représente au choix un groupe OH ou méthoxy, $R_4$ représente un groupe méthyle, $R_5$ représente un groupe méthyle, le chlore, un groupe $NO_2$ ou $NH_2$, $R_6$ représente l'hydrogène, un groupe méthyle, le chlore, un groupe $NO_2$ ou $NH_2$ et $R_7$ représente l'hydrogène ou un groupe méthyle.

6. Dérivés de l'homosérine de formule I selon la revendication 1, dans laquelle Ar représente un groupe alpha-naphtyle substitué, $R_1$ représente un groupe (alcoxy en C 1–C 2)-méthyle ou 2-tétrahydrofuryle, $R_2$ représente l'hydrogène, $R_3$ représente l'hydrogène ou un groupe méthyle, B représente au choix un groupe OH ou méthoxy, $R_8$ représente l'hydrogène ou un groupe 3-méthyle, $R_{12}$ représente un groupe méthyle, $NO_2$ ou $NH_2$ et $R_{13}$ représente l'hydrogène, un groupe méthyle, $NO_2$ ou $NH_2$.

7. Dérivés de l'homosérine de formule I selon la revendication 6, dans laquelle $R_{13}$ représente l'hydrogène, un groupe 4-$NO_2$ ou 4-$NH_2$.

8. Dérivés de l'homosérine de formule I selon la revendication 5, dans laquelle $R_1$ représente le groupe 2-tétrahydrofuryle.

9. Dérivés de l'homosérine de formule I selon la revendication 6, dans laquelle $R_1$ représente le groupe 2-tétrahydrofuryle.

10. L'ester méthylique de la N-(2,6-diméthylphényl)-N-méthoxyacéthyl-homosérine selon la revendication 1.

11. L'ester éthylique de la N-(2,6-diméthylphényl)-N-méthoxyacétyl-homosérine selon la revendication 1.

12. L'ester méthylique de la N-(2,3,6-triméthylphényl)-N-méthoxyacétyl-homosérine selon la revendication 1.

13. Le N-(2,6-diméthylphényl)-N-méthoxyacétyl-[4-(imidazole-1-yl)-carbonyloxy]-butyrate de méthyle selon la revendication 1.

14. L'ester méthylique de la N-(2-méthylnaphtyl)-N-méthoxyacétyl-homosérine selon la revendication 1.

15. L'ester méthylique de la N-(2-méthylnaphtyl)-N-(tétrahydrofurylcarbonyl)-homosérine selon la revendication 1.

16. L'ester méthylique de la N-(2-chloro-6-méthoxyphényl)-N-méthoxyacétyl-homosérine selon la revendication 1.

17. L'ester méthylique de la N-(2,6-diméthyl-3-chlorophényl)-N-méthoxy-acétyl-homosérine selon la revendication 1.

18. Le N-(2-méthylnaphtyl)-N-méthoxyacétyl-4-(N'-méthylcarbamoyloxy)-2-aminobutyrate de méthyle selon la revendication 1.

19. L'ester méthylique de la N-(2,3-diméthylnaphtyl)-N-méthoxyacétyl-homosérine selon la revendication 1.

20. L'ester méthylique de la N-(2-méthyl-6-nitrophényl)-N-méthoxyacétyl-homosérine selon la revendication 1.

21. Produit pour combattre et/ou prévenir une infestation par des microorganismes phytopathogènes, caractérisé en ce que l'un au moins de ces composants actifs est un composé de formule I selon la revendication 1, accompagné d'un ou plusieurs véhicules.

22. Produit selon la revendication 21, caractérisé en ce qu'il contient en tant que composant actif un composé de formule I selon la revendication 2.

23. Produit selon la revendication 21, caractérisé en ce qu'il contient en tant que composant actif un composé de formule I selon la revendication 3.

24. Produit selon la revendication 21, caractérisé en ce qu'il contient en tant que composant actif un composé de formule I selon la revendication 4.

25. Produit selon la revendication 21, caractérisé en ce qu'il contient en tant que composant actif un composé de formule I selon l'une des revendications 5 à 20.

26. Utilisation d'un composé de formule I selon la revendication 1, dans la lutte et/ou la prévention contre une infestation par des microorganismes phytopathogènes.

27. Utilisation selon la revendication 26 d'un composé de formule I selon la revendication 2.

28. Utilisation selon la revendication 26 d'un composé de formule I selon l'une des revendications 3 à 20.

29. Procédé de préparation des dérivés de l'homosérine de formule I selon la revendication 1, caractérisé en ce que l'on soumet un composé de formule II

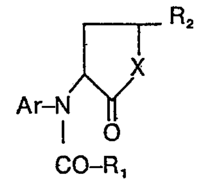

(II)

à ouverture alcaline du cycle dans un solvant polaire à l'aide d'un hydroxyde alcalin ou alcalino-terreux à une température de −10 à +100 °C, Ar, $R_1$, $R_2$ et X ayant les significations indiquées en référence à la formule I, et, si on le désire, on introduit ensuite l'un des substituants mentionnés en référence à $R_3$ ou B.

**Revendications pour l'Etat contractant: AT**

1. Produit pour combattre et/ou prévenir une infestation par des microorganismes phytopathogènes, caractérisé en ce qu'il contient, avec un ou plusieurs véhicules, au moins un composant actif consistant en un dérivé de l'homosérine de formule I

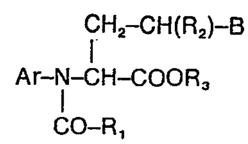

(I),

dans laquelle $R_1$ représente une chaîne aliphatique en C 2–C 6 éventuellement interrompue par un atome d'oxygène, ou un groupe 2-furyle, 2-tétrahydrofuryle, 1H-1,2,4-triazolylméthyle, 1-imidazolylméthyle, alcényle en C 2–C 4 ou cyclopropyle éventuellement substitué par des halogènes ou dans laquelle, lorsque B représente un halogène, $R_1$ représente un groupe halogénométhyle; $R_2$ représente l'hydrogène ou un groupe méthyle, $R_3$ représente l'hydrogène ou un groupe alkyle en C 1–C 4 et Ar représente un groupe

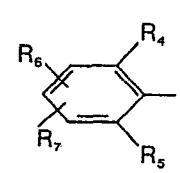

ou

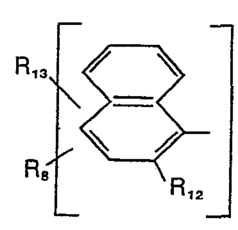

dans lequel $R_4$ reürésente un groupe alkyle en C 1–C 3, alcoxy en C 1–C 3 ou un halogène, $R_5$ représente un groupe alkyle en C 1–C 3, alcoxy en C 1–C 3, $NH_2$, un halogène ou un groupe $NO_2$, $R_6$ représente l'hydrogène, un groupe $NO_2$, $NH_2$, alkyle en C 1–C 3, alcoxy en C 1–C 3 ou un halogène, $R_7$ et $R_8$ représentent l'hydrogène ou des groupes méthyle, $R_{12}$ représente un groupe méthyle, $NO_2$ ou $NH_2$, $R_{13}$ représente l'hydrogène, un groupe méthyle, $NO_2$ ou $NH_2$ et B représente l'un des groupes –XH, –$XR_9$,

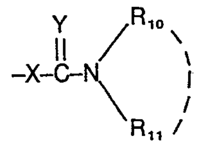

ou un halogène, X et Y représentant chacun, indépendamment l'un de l'autre, l'oxygène ou le soufre; $R_9$ représente un groupe alkyle en C 1–C 5 éventuellement substitué par des halogènes, des groupes alcoxy en C 1–C 3 ou alkylthio en C 1–C 3, $R_{10}$ représente l'hydrogène, un groupe méthyle ou éthyle, $R_{11}$ représente un groupe alkyle en C 1–C 5 éventuellement substitué par des halogènes ou un groupe phényle éventuellement substitué par des halogènes, des groupes méthyle, trifluorométhyle ou nitro ou bien $R_{10}$ et $R_{11}$ forment ensemble et avec l'atome d'azote auquel ils sont reliés un cycle imidazole ou 1,2,4-triazole.

2. Produit selon la revendication 1, pour lequel, dans la formule I, Ar représente

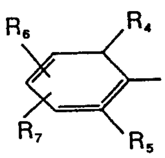

ou

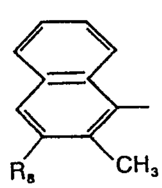

$R_1$, $R_2$, $R_3$, $R_4$ et B ont les significations indiquées dans la revendication 1 et $R_5$ représente un groupe alkyle en C 1–C 3, alcoxy en C 1–C 3 ou un halogène, $R_6$ représente l'hydrogène, un groupe alkyle en C 1–C 3, alcoxy en C 1–C 3 ou un halogène et $R_7$ et $R_8$ représentent l'hydrogène ou des groupes méthyle.

3. Produit selon la revendication 1, pour lequel, dans la formule I, $R_1$ représente un groupe (alcoxy en C 1–C 3)-méthyle, 2-furyle ou 2-tétrahydrofuryle, $R_2$ et $R_3$ représentent l'hydrogène, B représente OH ou SH, $R_4$ représente un groupe alkyle en C 1–C 3, alcoxy en C 1–C 3 ou un halogène, $R_5$ représente un groupe alkyle en C 1–C 3, alcoxy en C 1–C 3, un halogène ou un groupe nitro, $R_6$ représente l'hydrogène ou un groupe alkyle en C 1–C 3, $R_7$ représente l'hydrogène ou un groupe méthyle et Ar un groupe phényle substitué.

4. Produit selon la revendication 1, pour lequel, dans la formule I, $R_1$ représente un groupe (alcoxy en C 1–C 3)-méthyle, 2-furyle ou 2-tétrahydrofuryle, $R_2$ représente l'hydrogène, $R_3$ représente un groupe alkyle en C 1–C 3, B représente $OR_9$, $R_9$ représente un groupe alkyle en C 1–C 3 éventuellement substitué par des halogènes, $R_4$ représente un groupe alkyle en C 1–C 3, alcoxy en C 1–C 3 ou un halogène, $R_5$ représente un groupe alkyle en C 1–C 3, alcoxy en C 1–C 3 ou un halogène, $R_6$ représente l'hydrogène ou un groupe alkyle en C 1–C 3, $R_7$ représente l'hydrogène ou un groupe méthyle et Ar représente un groupe phényle substitué.

5. Produit selon la revendication 1, pour lequel, dans la formule I, Ar représente un groupe phényle substitué, $R_1$ représente un groupe (alcoxy en C 1–C 2)-méthyle ou 2-tétrahydrofuryle, $R_2$ représente l'hydrogène, $R_3$ représente l'hydrogène ou un groupe méthyle, B représente au choix un groupe OH ou méthoxy, $R_4$ représente un groupe méthyle, $R_5$ représente un groupe méthyle, le chlore, un groupe $NO_2$ ou $NH_2$, $R_6$ représente l'hydrogène, un groupe méthyle, le chlore, un groupe $NO_2$ ou $NH_2$ et $R_7$ représente l'hydrogène ou un groupe méthyle.

6. Produit selon la revendication 1, pour lequel, dans la formule I, Ar représente un groupe alphanaphtyle substitué, $R_1$ représente un groupe (alcoxy en C 1–C 2)-méthyle ou 2-tétrahydrofuryle, $R_2$ représente l'hydrogène, $R_3$ représente l'hydrogène ou un groupe méthyle, B représente au choix un groupe OH ou méthoxy, $R_8$ représente l'hydrogène ou un groupe 3-méthyle, $R_{12}$ représente un groupe méthyle, $NO_2$ ou $NH_2$ et $R_{13}$ représente l'hydrogène, un groupe méthyle, $NO_2$ ou $NH_2$.

7. Produit selon la revendication 6, pour lequel, dans la formule I, $R_{13}$ représente l'hydrogène, un groupe 4-$NO_2$ ou 4-$NH_2$.

8. Produit selon la revendication 5, pour lequel, dans la formule I, $R_1$ représente le groupe 2-tétrahydrofuryle.

9. Produit selon la revendication 6, pour lequel, dans la formule I, $R_1$ représente un groupe 2-tétrahydrofuryle.

10. Produit selon la revendication 1, contenant l'ester méthylique de la
N-(2,6-diméthylphényl)-N-méthoxyacétyl-homosérine.

11. Produit selon la revendication 1, contenant l'ester éthylique de la
N-(2,6-diméthylphényl)-N-méthoxyacétyl-homosérine.

12. Produit selon la revendication 1, contenant l'ester méthylique de la
N-(2,3,6-triméthylphényl)-N-méthoxyacétyl-homosérine.

13. Produit selon la revendication 1, contenant du
N-(2,6-diméthylphényl)-N-méthoxyacétyl-[4-(imidazole-1-yl)-carbonyloxy]-butyrate de méthyle.

14. Produit selon la revendication 1, contenant l'ester méthylique de la
N-(2-méthylnaphtyl)-N-méthoxyacétyl-homosérine.

15. Produit selon la revendication 1, contenant l'ester méthylique de la
N-(2-méthylnaphthyl)-N-(tétrahydrofurylcarbonyl)-homosérine.

16. Produit selon la revendication 1, contenant l'ester méthylique de la
N-(2-chloro-6-méthoxyphényl)-N-méthoxyacétyl-homosérine.

17. Produit selon la revendication 1, contenant l'ester méthylique de la N-(2,6-diméthyl-3-chlorophényl)-N-méthoxyacétyl-homosérine.

18. Produit selon la revendication 1, contenant du N-(2-méthylnaphtyl)-N-méthoxyacétyl-4-(N'-méthylcarbamoyloxy)-2-aminobutyrate de méthyle.

19. Produit selon la revendication 1, contenant l'ester méthylique de la N-(2,3-diméthylnaphtyl)-M-méthoxyacétyl-homosérine.

20. Produit selon la revendication 1, contenant l'ester méthylique de la N-(2-méthyl-6-nitrophényl)-N-méthoxyacétyl-homo-sérine.

21. Utilisation d'un composé de formule I selon la revendication 1, dans la lutte ou la prévention contre une infestation par les microorganismes phytopathogènes.

22. Utilisation selon la revendication 21 d'un composé de formule I selon la revendication 2.

23. Utilisation selon la revendication 21 d'un composé de formule I selon l'une des revendications 3 à 20.

24. Procédé de préparation des dérivés de l'homosérine de formule I selon la revendication 1, caractérisé en ce que l'on soumet un composé de formule II

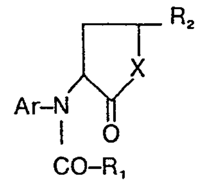

(II)

à ouverture alcaline du cycle dans un solvant polaire à l'aide d'un hydroxyde de métal alcalin ou alcalinoterreux à une température de $-10$ à $+100\,°C$, Ar, $R_1$, $R_2$ et X ayant les significations indiquées en référence à la formule I et, si on le désire, on introduit ensuite l'un des substituants mentionnés en référence à $R_3$ ou B.